# EUROPEAN PATENT SPECIFICATION

(11) **EP 1 843 789 B1**
(45) Date of publication and mention of the grant of the patent: **30.03.2011**
(21) Application number: 06720055.0
(22) Date of filing: 01.02.2006
(51) Int. Cl.: A61K 39/395, A61K 39/17, A61P 11/00, A61P 17/02, A61P 43/00, A61P 1/16, A61K 31/7088, A61K 38/20

(54) **COMPOSITIONS AND METHODS FOR TREATING FIBROTIC DISORDERS**
ZUSAMMENSETZUNGEN UND VERFAHREN ZUR BEHANDLUNG FIBROTISCHER STÖRUNGEN
COMPOSITIONS ET PROCÉDÉS POUR LE TRAITEMENT DE TROUBLES FIBROTIQUES

(30) Priority: 01.02.2005 US 649287 P
(43) Date of publication of application: 17.10.2007
(73) Proprietor: Amgen Inc., Thousand Oaks, CA 91320-1799 (US)
(72) Inventor: COMEAU, Michael, R., Bainbridge Island, WA 98110 (US); FITZPATRICK, David, R., Fort Collins, CO 80526 (US)
(74) Representative: Cornish, Kristina Victoria Joy
(86) International application number: PCT/US2006/003519
(87) International publication number: WO 2006/083947

(56) References cited:
- WO-A-02/00724
- WO-A-95/00103
- WO-A-98/36061
- WO-A-03/065985
- WO-A-2006/023791
- US-A1- 2002 146 819
- US-A1- 2003 181 360
- US-A1- 2005 249 712
- LEVIN S D ET AL: "Thymic stromal lymphopoietin: a cytokine that promotes the development of IgM + B cells in vitro and signals via a novel mechanism" JOURNAL OF IMMUNOLOGY, THE WILLIAMS AND WILKINS CO. BALTIMORE, US, vol. 162, no. 2, 15 January 1999 (1999-01-15), pages 677-683, XP002193894 ISSN: 0022-1767
- BLYTH D I ET AL: "Airway subepithelial fibrosis in a murine model of atopic asthma: suppression by dexamethasone or anti-interleukin-5 antibody." AMERICAN JOURNAL OF RESPIRATORY CELL AND MOLECULAR BIOLOGY. AUG 2000, vol. 23, no. 2, August 2000 (2000-08), pages 241-246, XP002386170 ISSN: 1044-1549
- ONG C ET AL: "Anti-IL-4 treatment prevents dermal collagen deposition in the tight-skin mouse model of scleroderma." EUROPEAN JOURNAL OF IMMUNOLOGY. SEP 1998, vol. 28, no. 9, September 1998 (1998-09), pages 2619-2629, XP002386171 ISSN: 0014-2980

### SEQUENCE LISTING

<110> AMGEN INC.
   Comeau, Michael R.
   Fitzpatrick, David R.
<120> COMPOSITIONS AND METHODS FOR TREATING FIBROTIC DISORDERS
<130> A-958 (WO)
<140> to be assigned
   <141> 2006-02-01
<150> US 60/649,287
   <151> 2005-02-01
<160> 4
<170> PatentIn version 3.3
<210> 1
   <211> 743
   <212> DNA
   <213> Homo sapiens
<220>
   <221> CDS
   <222> (200)..(676)
<400> 1
   gcagccagaa agctctggag catcagggag actccaactt aaggcaacag catgggtgaa     60
   taagggcttc ctgtggactg gcaatgagag gcaaaacctg gtgcttgagc actggcccct    120
   aaggcaggcc ttacagatct cttacactcg tggtgggaag agtttagtgt gaaactgggg    180
   tggaattggg tgtccacgt atg ttc cct ttt gcc tta cta tat gtt ctg tca     232
                        Met Phe Pro Phe Ala Leu Leu Tyr Val Leu Ser
                        1               5                   10
   gtt tct ttc agg aaa atc ttc atc tta caa ctt gta ggg ctg gtg tta      280
   Val Ser Phe Arg Lys Ile Phe Ile Leu Gln Leu Val Gly Leu Val Leu
               15                  20                  25
   act tac gac ttc act aac tgt gac ttt gag aag att aaa gca gcc tat      328
   Thr Tyr Asp Phe Thr Asn Cys Asp Phe Glu Lys Ile Lys Ala Ala Tyr
           30                  35                  40
   ctc agt act att tct aaa gac ctg att aca tat atg agt ggg acc aaa      376
   Leu Ser Thr Ile Ser Lys Asp Leu Ile Thr Tyr Met Ser Gly Thr Lys
       45                  50                  55
   agt acc gag ttc aac aac acc gtc tct tgt agc aat cgg cca cat tgc      424
   Ser Thr Glu Phe Asn Asn Thr Val Ser Cys Ser Asn Arg Pro His Cys
   60                  65                  70                  75
   ctt act gaa atc cag agc cta acc ttc aat ccc acc gcc ggc tgc gcg      472
   Leu Thr Glu Ile Gln Ser Leu Thr Phe Asn Pro Thr Ala Gly Cys Ala
                   80                  85                  90
   tcg ctc gcc aaa gaa atg ttc gcc atg aaa act aag gct gcc tta gct      520
   Ser Leu Ala Lys Glu Met Phe Ala Met Lys Thr Lys Ala Ala Leu Ala
               95                  100                 105
   atc tgg tgc cca ggc tat tcg gaa act cag ata aat gct act cag gca      568
   Ile Trp Cys Pro Gly Tyr Ser Glu Thr Gln Ile Asn Ala Thr Gln Ala
           110                 115                 120
   atg aag aag agg aga aaa agg aaa gtc aca acc aat aaa tgt ctg gaa      616
   Met Lys Lys Arg Arg Lys Arg Lys Val Thr Thr Asn Lys Cys Leu Glu
       125                 130                 135
   caa gtg tca caa tta caa gga ttg tgg cgt cgc ttc aat cga cct tta      664
   Gln Val Ser Gln Leu Gln Gly Leu Trp Arg Arg Phe Asn Arg Pro Leu
   140                 145                 150                 155
   ctg aaa caa cag taaaccatct ttattatggt catatttcac agcccaaaat          716
   Leu Lys Gln Gln
   aaatcatctt tattaagtaa aaaaaaa                                        743
<210> 2
   <211> 159
   <212> PRT
   <213> Homo sapiens
<400> 2
   Met Phe Pro Phe Ala Leu Leu Tyr Val Leu Ser Val Ser Phe Arg Lys
   1               5                   10                  15
   Ile Phe Ile Leu Gln Leu Val Gly Leu Val Leu Thr Tyr Asp Phe Thr
               20                  25                  30
   Asn Cys Asp Phe Glu Lys Ile Lys Ala Ala Tyr Leu Ser Thr Ile Ser
           35                  40                  45
   Lys Asp Leu Ile Thr Tyr Met Ser Gly Thr Lys Ser Thr Glu Phe Asn
       50                  55                  60
   Asn Thr Val Ser Cys Ser Asn Arg Pro His Cys Leu Thr Glu Ile Gln
   65                  70                  75                  80
   Ser Leu Thr Phe Asn Pro Thr Ala Gly Cys Ala Ser Leu Ala Lys Glu
                   85                  90                  95
   Met Phe Ala Met Lys Thr Lys Ala Ala Leu Ala Ile Trp Cys Pro Gly
               100                 105                 110
   Tyr Ser Glu Thr Gln Ile Asn Ala Thr Gln Ala Met Lys Lys Arg Arg
           115                 120                 125
   Lys Arg Lys Val Thr Thr Asn Lys Cys Leu Glu Gln Val Ser Gln Leu
       130                 135                 140
   Gln Gly Leu Trp Arg Arg Phe Asn Arg Pro Leu Leu Lys Gln Gln
   145                 150                 155
<210> 3
   <211> 1116
   <212> DNA
   <213> Homo sapiens
<220>
   <221> CDS
   <222> (1)..(1116)
<220>
   <221> sig_peptide
   <222> (1)..(66)
<220>
   <221> misc_feature
   <222> (694)..(756)
<400> 3
   atg ggg cgg ctg gtt ctg ctg tgg gga gct gcc gtc ttt ctg ctg gga      48
   Met Gly Arg Leu Val Leu Leu Trp Gly Ala Ala Val Phe Leu Leu Gly
   1               5                   10                  15
   ggc tgg atg gct ttg ggg caa gga gga gca gca gaa gga gta cag att      96
   Gly Trp Met Ala Leu Gly Gln Gly Gly Ala Ala Glu Gly Val Gln Ile
               20                  25                  30
   cag atc atc tac ttc aat tta gaa acc gtg cag gtg aca tgg aat gcc     144
   Gln Ile Ile Tyr Phe Asn Leu Glu Thr Val Gln Val Thr Trp Asn Ala
           35                  40                  45
   agc aaa tac tcc agg acc aac ctg act ttc cac tac aga ttc aac ggt     192
   Ser Lys Tyr Ser Arg Thr Asn Leu Thr Phe His Tyr Arg Phe Asn Gly
       50                  55                  60
   gat gag gcc tat gac cag tgc acc aac tac ctt ctc cag gaa ggt cac     240
   Asp Glu Ala Tyr Asp Gln Cys Thr Asn Tyr Leu Leu Gln Glu Gly His
   65                  70                  75                  80
   act tca ggg tgc ctc cta gac gca gag cag cga gac gac att ctc tat     288
   Thr Ser Gly Cys Leu Leu Asp Ala Glu Gln Arg Asp Asp Ile Leu Tyr
                   85                  90                  95
   ttc tcc atc agg aat ggg acg cac ccc gtt ttc acc gca agt cgc tgg     336
   Phe Ser Ile Arg Asn Gly Thr His Pro Val Phe Thr Ala Ser Arg Trp
               100                 105                 110
   atg gtt tat tac ctg aaa ccc agt tcc ccg aag cac gtg aga ttt tcg     384
   Met Val Tyr Tyr Leu Lys Pro Ser Ser Pro Lys His Val Arg Phe Ser
           115                 120                 125
   tgg cat cag gat gca gtg acg gtg acg tgt tct gac ctg tcc tac ggg      432
   Trp His Gln Asp Ala Val Thr Val Thr Cys Ser Asp Leu Ser Tyr Gly
       130                 135                 140
   gat ctc ctc tat gag gtt cag tac cgg agc ccc ttc gac acc gag tgg      480
   Asp Leu Leu Tyr Glu Val Gln Tyr Arg Ser Pro Phe Asp Thr Glu Trp
   145                 150                 155                 160
   cag tcc aaa cag gaa aat acc tgc aac gtc acc ata gaa ggc ttg gat      528
   Gln Ser Lys Gln Glu Asn Thr Cys Asn Val Thr Ile Glu Gly Leu Asp
                   165                 170                 175
   gcc gag aag tgt tac tct ttc tgg gtc agg gtg aag gct atg gag gat      576
   Ala Glu Lys Cys Tyr Ser Phe Trp Val Arg Val Lys Ala Met Glu Asp
               180                 185                 190
   gta tat ggg cca gac aca tac cca agc gac tgg tca gag gtg aca tgc      624
   Val Tyr Gly Pro Asp Thr Tyr Pro Ser Asp Trp Ser Glu Val Thr Cys
           195                 200                 205
   tgg cag aga ggc gag att cgg gat gcc tgt gca gag aca cca acg cct      672
   Trp Gln Arg Gly Glu Ile Arg Asp Ala Cys Ala Glu Thr Pro Thr Pro
       210                 215                 220
   ccc aaa cca aag ctg tcc aaa ttt att tta att tcc agc ctg gcc atc      720
   Pro Lys Pro Lys Leu Ser Lys Phe Ile Leu Ile Ser Ser Leu Ala Ile
   225                 230                 235                 240
   ctt ctg atg gtg tct ctc ctc ctt ctg tct tta tgg aaa tta tgg aga      768
   Leu Leu Met Val Ser Leu Leu Leu Leu Ser Leu Trp Lys Leu Trp Arg
                   245                 250                 255
   gtg aag aag ttt ctc att ccc agc gtg cca gac ccg aaa tcc atc ttc      816
   Val Lys Lys Phe Leu Ile Pro Ser Val Pro Asp Pro Lys Ser Ile Phe
               260                 265                 270
   ccc ggg ctc ttt gag ata cac caa ggg aac ttc cag gag tgg atc aca      864
   Pro Gly Leu Phe Glu Ile His Gln Gly Asn Phe Gln Glu Trp Ile Thr
           275                 280                 285
   gac acc cag aac gtg gcc cac ctc cac aag atg gca ggt gca gag caa      912
   Asp Thr Gln Asn Val Ala His Leu His Lys Met Ala Gly Ala Glu Gln
       290                 295                 300
   gaa agt ggc ccc gag gag ccc ctg gta gtc cag ttg gcc aag act gaa      960
   Glu Ser Gly Pro Glu Glu Pro Leu Val Val Gln Leu Ala Lys Thr Glu
   305                 310                 315                 320
   gcc gag tct ccc agg atg ctg gac cca cag acc gag gag aaa gag gcc     1008
   Ala Glu Ser Pro Arg Met Leu Asp Pro Gln Thr Glu Glu Lys Glu Ala
                   325                 330                 335
   tct ggg gga tcc ctc cag ctt ccc cac cag ccc ctc caa ggc ggt gat     1056
   Ser Gly Gly Ser Leu Gln Leu Pro His Gln Pro Leu Gln Gly Gly Asp
               340                 345                 350
   gtg gtc aca atc ggg ggc ttc acc ttt gtg atg aat gac cgc tcc tac     1104
   Val Val Thr Ile Gly Gly Phe Thr Phe Val Met Asn Asp Arg Ser Tyr
           355                 360                 365
   gtg gcg ttg tga                                                     1116
   Val Ala Leu
   370
<210> 4
   <211> 371
   <212> PRT
   <213> Homo sapiens
<400> 4
   Met Gly Arg Leu Val Leu Leu Trp Gly Ala Ala Val Phe Leu Leu Gly
   1               5                   10                  15
   Gly Trp Met Ala Leu Gly Gln Gly Gly Ala Ala Glu Gly Val Gln Ile
               20                  25                  30
   Gln Ile Ile Tyr Phe Asn Leu Glu Thr Val Gln Val Thr Trp Asn Ala
           35                  40                  45
   Ser Lys Tyr Ser Arg Thr Asn Leu Thr Phe His Tyr Arg Phe Asn Gly
       50                  55                  60
   Asp Glu Ala Tyr Asp Gln Cys Thr Asn Tyr Leu Leu Gln Glu Gly His
   65                  70                  75                  80
   Thr Ser Gly Cys Leu Leu Asp Ala Glu Gln Arg Asp Asp Ile Leu Tyr
                   85                  90                  95
   Phe Ser Ile Arg Asn Gly Thr His Pro Val Phe Thr Ala Ser Arg Trp
               100                 105                 110
   Met Val Tyr Tyr Leu Lys Pro Ser Ser Pro Lys His Val Arg Phe Ser
           115                 120                 125
   Trp His Gln Asp Ala Val Thr Val Thr Cys Ser Asp Leu Ser Tyr Gly
       130                 135                 140
   Asp Leu Leu Tyr Glu Val Gln Tyr Arg Ser Pro Phe Asp Thr Glu Trp
   145                 150                 155                 160
   Gln Ser Lys Gln Glu Asn Thr Cys Asn Val Thr Ile Glu Gly Leu Asp
                   165                 170                 175
   Ala Glu Lys Cys Tyr Ser Phe Trp Val Arg Val Lys Ala Met Glu Asp
               180                 185                 190
   Val Tyr Gly Pro Asp Thr Tyr Pro Ser Asp Trp Ser Glu Val Thr Cys
           195                 200                 205
   Trp Gln Arg Gly Glu Ile Arg Asp Ala Cys Ala Glu Thr Pro Thr Pro
       210                 215                 220
   Pro Lys Pro Lys Leu Ser Lys Phe Ile Leu Ile Ser Ser Leu Ala Ile
   225                 230                 235                 240
   Leu Leu Met Val Ser Leu Leu Leu Leu Ser Leu Trp Lys Leu Trp Arg
                   245                 250                 255
   Val Lys Lys Phe Leu Ile Pro Ser Val Pro Asp Pro Lys Ser Ile Phe
               260                 265                 270
   Pro Gly Leu Phe Glu Ile His Gln Gly Asn Phe Gln Glu Trp Ile Thr
           275                 280                 285
   Asp Thr Gln Asn Val Ala His Leu His Lys Met Ala Gly Ala Glu Gln
       290                 295                 300
   Glu Ser Gly Pro Glu Glu Pro Leu Val Val Gln Leu Ala Lys Thr Glu
   305                 310                 315                 320
   Ala Glu Ser Pro Arg Met Leu Asp Pro Gln Thr Glu Glu Lys Glu Ala
                   325                 330                 335
   Ser Gly Gly Ser Leu Gln Leu Pro His Gln Pro Leu Gln Gly Gly Asp
               340                 345                 350
   Val Val Thr Ile Gly Gly Phe Thr Phe Val Met Asn Asp Arg Ser Tyr
           355                 360                 365
   Val Ala Leu
       370

## Description

### FIELD OF THE INVENTION

This invention relates to compositions and methods for treating fibrotic disorders.

### BACKGROUND OF THE INVENTION

The process of tissue repair as a part of wound healing involves two phases. The first phase is the regenerative phase, in which injured cells are replaced by cells of the same type. The second phase is the formation of fibrous tissues, also called fibroplasia or fibrosis, in which connective tissue replaces normal parenchymal tissues. The tissue repair process can become pathogenic if the fibrosis phase continues unchecked, leading to extensive tissue remodeling and the formation of permanent scar tissue (Wynn, Nature Rev. Immunol. 4, 583 (2004)).

It has been estimated that up to 45% of deaths in the United States can be attributed to fibroproliferative diseases, which can affect many tissues and organ systems. (Wynn, *supra,* at 595 (2004)). Major organ fibrotic diseases include interstitial lung disease (ILD), characterized by pulmonary inflammation and fibrosis. ILD is known to have a number of causes such as sarcoidosis, silicosis, collagen vascular diseases, and systemic scleroderma. However, idiopathic pulmonary fibrosis, a common type of ILD, has no known cause. Other organ fibrotic disorders include liver cirrhosis, liver fibrosis resulting from chronic hepatitis B or C infection, kidney disease, heart disease, and eye diseases including macular degeneration and retinal and vitreal retinopathy. Fibroproliferative disorders also include systemic and local scleroderma, keloids and hypertrophic scars, atherosclerosis, and restenosis. Additional fibroproliferative diseases include excessive scarring resulting from surgery, chemotherapeutic drug-induced fibrosis, radiation-induced fibrosis, and injuries and burns (Wynn, *supra,* page 585).

Currently, treatments are available for fibrotic disorders including general immunosuppressive drugs such as corticosteroids, and other anti-inflammatory treatments. However, the mechanisms involved in regulation of fibrosis appear to be distinctive from those of inflammation, and anti-inflammatory therapies are not always effective in reducing or preventing fibrosis (Wynn, *supa,* page 591). WO98/36061 relates to reducing fibrosis and/or scarring by inhibiting IL-6 receptor mediated activity. Therefore, a need remains for developing treatments to reduce and prevent fibrosis and control fibrotic disorders.

The present invention addresses this need and provides methods and compositions for preventing or reducing fibrosis associated with fibrotic disorders.

### SUMMARY OF THE INVENTION

The present invention provides methods for modulating fibroblast accumulation and collagen deposition in a tissue by modulating the amount or activity of the cytokine thymic stromal lymphopoietin (TSLP) in the tissue. In one aspect, the present invention provides a TSLP antagonist for use in reducing or preventing fibrosis in a subject suffering from a fibrotic disorder wherein the antagonist binds to thymic stromal lymphopoietin or to thymic stromal lymphopoetin receptor and is selected from the group consisting of an antibody, a peptide or polypeptide. In another aspect, the present invention provides for the use of at least one TSLP antagonist in the preparation of a medicament for the prevention or treatment of a fibrotic disorder in a subject suffering from such a disorder. The invention further provides a pharmaceutical composition for preventing or reducing fibrosis in a subject suffering from a fibrotic disorder comprising a therapeutically effective dosage of at least one antagonist to TSLP in admixture with a pharmaceutically acceptable carrier. The Fibrotic disorders include, but are not limited to, scleroderma, interstitial lung disease (ILD), idiopathic pulmonary fibrosis (IPF), liver fibrosis resulting from chronic hepatitis B or C infection, radiation-induced fibrosis, and fibrosis arising from wound healing.

In one embodiment the TSLP antagonist is a TSLP ligand binding agent capable of binding to TSLP and reducing or blocking its activity. These antagonists include, but are not limited to, antagonistic antibodies, peptide or polypeptide binding agents, soluble TSLP receptors (TSLPR), soluble interleukin 7 receptor alpha (IL-7 R α)/TSLPR heterodimer receptors (heterodimer) antagonists. The antagonistic antibodies include, but are not limited to, fully human, humanized, chimeric, single chain antibodies, and antibody fragments. The peptide or polypeptide binding agents, soluble receptor and soluble heterodimer receptor antagonists may further comprise Fc domains or other multimerizing components, or carrier molecules such as PEG.

In another embodiment, the TSLP antagonist is a TSLPR antagonist. TSLPR antagonists include antagonists which bind to the TSLP receptor, and antagonists which bind to the IL- 7Rα/TSLPR heterodimer. These antagonists include, but are not limited to, antagonistic antibodies which bind to TSLPR; antagonistic antibodies which bind to the heterodimer, soluble ligands which bind to the TSLPR; soluble ligands which bind to the heterodimer. The antagonistic antibodies include, but are not limited to, human, humanized, chimeric, and single-chain antibodies, and antibody fragments. The soluble ligand may further comprise Fc domains or other multimerizing components, or carrier molecules such as PEG.

In another embodiment, the methods and compositions of the present invention further comprise at least one additional antagonist to one or more cytokine, growth factor, or chemokine which promotes fibrosis. These profibrotic factors include, but are not limited to, transforming growth factor β (TGF-β), interleukin- 4 (IL-4), interleukin-5 (IL-5), interleukin-9 (IL-9), interleukin-13 (IL-13), granulocyte/macrophage-colony stimulating factor (GM-CSF), tumor necrosis factor alpha (TNF-α), interleukin-1 beta (IL-I β), connective tissue growth factor (CTGF), interleukin-6 (IL-6), oncostatin M (OSM), platelet derived growth factor (PDGF), monocyte chemotactic protein 1(CCL2/MCP-1), and pulmonary and activation-regulated chemokine (CCL1 8/PARC).

### BRIEF DESCRIPTION OF THE FIGURES

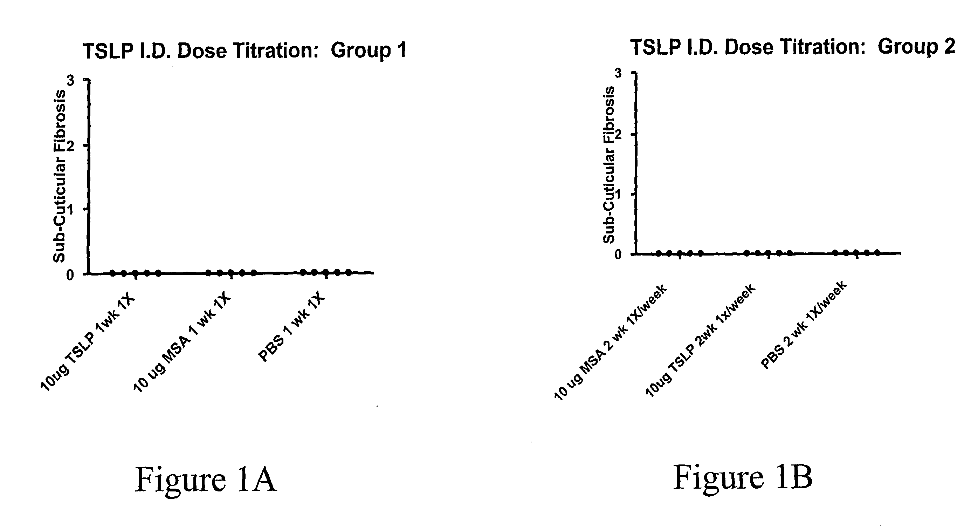

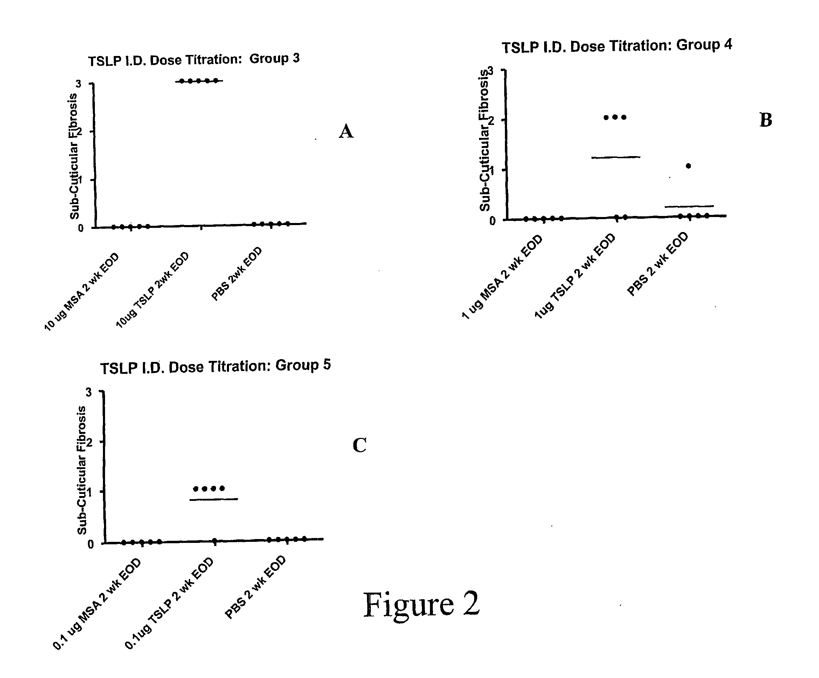

Figures 1A and 1B, and Figure 2A, 2B, and 2C show the results of injecting five groups of Balb/c mice intradermally with varying dosages ofTSLP and a negative control MSA (mouse serum albumin) once a week for 1 week (Figure 1A, Group 1), once a week for 2 weeks (Figure 1B, Group 2); and three times a week for two weeks in Figure 2A (Group 3), 2B (Group 4) and 2C (Group 5). Figure 1A (Group 1) shows no subcuticular fibrosis induced from a single injection of 10 ug TSLP for one week; MSA alone; and PBS alone. Figure 1B (Group 2) shows no subcuticular fibrosis induced from a single injection on each of two weeks (2 total injections) of 10 ug TSLP; MSA alone, and PBS alone. Figure 2A (Group 3) shows subcuticular fibrosis scored at level 3 for 10 ug TSLP when injected three times a week for 2 weeks, but no fibrosis for MSA alone, and PBS alone. Figure 2B (Group 4) shows fibrosis scored at level 2 for 1 ug TSLP when injected three times a week for 2 weeks, but no fibrosis for MSA alone, and none for PBS alone with the exception of one animal showing fibrosis at level 1 for PBS alone. Figure 2C (Group 5) shows fibrosis scored at level 1 for lug TSLP when injected three times a week for 2 weeks, but no fibrosis for MSA alone or PBS alone.

### DETAILED DESCRIPTION OF THE INVENTION

The present invention provides a TSLP antagonist for use in modulating fibroblast accumulation and collagen deposition in a tissue by modulating the amount or activity of the cytokine thymic stromal lymphopoietin (TSLP) in the tissue. TSLP has been found to induce fibroblast accumulation and collagen deposition characteristic of fibrotic disorders in animals. In another aspect, the present invention provides an antagonist to TSLP for use in reducing or preventing fibrosis in a subject suffering from a fibrotic disorder. In another aspect, the present invention provides for the use of at least one TSLP antagonist in the preparation of a medicament for the prevention or treatment of a fibrotic disorder in a subject suffering from such a disorder. In another aspect, the present invention provides a pharmaceutical composition for preventing or reducing fibrosis in a subject comprising a therapeutically effective dosage of at least one antagonist to TSLP in admixture with a pharmaceutically acceptable carrier.

As used herein the term "fibroproliferative disease" or "fibrotic disease or disorder" refers to conditions involving fibrosis in one or more tissues. As used herein the term "fibrosis" refers to the formation of fibrous tissue as a reparative or reactive process, rather than as a normal constituent of an organ or tissue. Fibrosis is characterized by fibroblast accumulation and collagen deposition in excess of normal deposition in any particular tissue. As used herein the term "fibrosis" is used synonymously with "fibroblast accumulation and collagen deposition". Fibroblasts are connective tissue cells, which are dispersed in connective tissue throughout the body. Fibroblasts secrete a nonrigid extracellular matrix containing type I and/or type III collagen. In response to an injury to a tissue, nearby fibroblasts migrate into the wound, proliferate, and produce large amounts of collagenous extracellular matrix. Collagen is a fibrous protein rich in glycine and proline that is a major component of the extracellular matrix and connective tissue, cartilage, and bone. Collagen molecules are triple-stranded helical structures called α-chains, which are wound around each other in a ropelike helix. Collagen exists in several forms or types; of these, type I, the most common, is found in skin, tendon, and bone; and type III is found in skin, blood vessels, and internal organs.

Fibrotic disorders include, but are not limited to, systemic and local scleroderma, keloids and hypertrophic scars, atherosclerosis, restinosis, pulmonary inflammation and fibrosis, idiopathic pulmonary fibrosis, liver cirrhosis, fibrosis as a result of chronic hepatitis B or C infection, kidney disease, heart disease resulting from scar tissue, and eye diseases such as macular degeneration, and retinal and vitreal retinopathy. Additional fibrotic diseases include fibrosis resulting from chemotherapeutic drugs, radiation-induced fibrosis, and injuries and burns.

Scleroderma is a fibrotic disorder characterized by a thickening and induration of the skin caused by the overproduction of new collagen by fibroblasts in skin and other organs. Scleroderma may occur as a local or systemic disease. Systemic scleroderma may affect a number of organs. Systemic sclerosis is characterized by formation of hyalinized and thickened collagenous fibrous tissue, with thickening of the skin and adhesion to underlying tissues, especially of the hands and face. The disease may also be characterized by dysphagia due to loss of peristalsis and submucosal fibrosis of the esophagus, dyspnea due to pulmonary fibrosis, myocardial fibrosis, and renal vascular changes. (Stedman's Medical Dictionary, 26th Edition, Williams & Wilkins, 1995)). Pulmonary fibrosis affects 30 to 70% of scleroderma patients, often resulting in restrictive lung disease (Atamas et al. Cytokine and Growth Factor Rev 14: 537-550 (2003)).

Idiopathic pulmonary fibrosis is a chronic, progressive and usually lethal lung disorder, thought to be a consequence of a chronic inflammatory process (Kelly et al., Curr Pharma Design 9: 39-49 (2003)). The causes of this disease are not yet known.

As used herein the term "subject" refers to animals including mammals including humans. The term "mammal" includes primates, domesticated animals including dogs, cats, sheep, cattle, goats, pigs, mice, rats, rabbits, guinea pigs, captive animals such as zoo animals, and wild animals. As used herein the term "tissue" refers to an organ or set of specialized cells such as skin tissue, lung tissue, kidney tissue, and other types of cells.

### TSLP

Thymic stromal lymphopoietin (TSLP) refers to a four α-helical bundle type I cytokine which is a member of the IL-2 family but most closely related to IL-7. Cytokines are low molecular weight regulatory proteins secreted in response to certain stimuli, which act on receptors on the membrane of target cells. Cytokines regulate a variety of cellular responses. Cytokines are generally described in references such as Cytokines, A. Mire-Sluis and R. Thorne, ed., Academic Press, New York, (1998).

TSLP was originally cloned from a murine thymic stromal cell line (Sims et al J. Exp. Med 192 (5), 671-680 (2000)), and found to support early B and T cell development. Human TSLP was later cloned and found to have a 43 percent identity in amino acid sequence to the murine homolog (Quentmeier et al. Leukemia 15, 1286-1292 (2001), and U.S. Patent No: 6,555,520). The polynucleotide and amino acid sequence of human TSLP are presented in SEQ ID NO: 1 and 2 respectively. TSLP was found to bind with low affinity to a receptor chain from the hematopoietin receptor family called TSLP receptor (TSLPR), which is described in U.S. Patent application No: 09/895,945 (publication No: 2002/0068323) (SEQ ID NO: 4 and 5). The polynucleotide sequence encoding human TSLPR is presented as SEQ ID NO: 3 of the present application, and the amino acid sequence is presented as SEQ ID NO: 4 of the present application respectively. The soluble domain of the TSLPR is approximately amino acids 25 through 231 of SEQ ID NO: 4. TSLP binds with high affinity to a heterodimeric complex of TSLPR and the interleukin 7 receptor alpha IL-7Rα (Park et al., J. Exp. Med 192:5 (2000), U.S. Patent application No. 09/895,945, publication number U.S. 2002/0068323). The sequence of IL-7 receptor α is shown in Figure 2 of U.S. Patent No. 5,264,416. The sequence of the soluble domain of the IL-7 receptor α is amino acid 1 to 219 of Figure 2 in U.S. Patent No: 5,264,416.

Human TSLP can also be expressed in modified form, in which a furin cleavage site has been removed through modification of the amino acid sequence, as described in PCT patent application publication WO 03/032898. Modified TSLP retains activity but the full length sequence is more easily expressed in microbial or mammalian cells.

TSLP is produced in human epithelial cells including skin, bronchial, tracheal, and airway epithelial cells, keratinocytes, stromal and mast cells, smooth muscle cells, and lung and dermal fibroblasts, as determined by quantitative mRNA analysis (Soumelis et al, Nature Immunol. 3 (7) 673-680 (2002)). Both murine and human TSLP are involved in promoting allergic inflammation. Soumelis et al, *supra* reported that the TSLP heterodimer receptor complex is expressed on human CD11c+ dendritic cells (DC cells). Dendritic cell culture experiments have shown that TSLP binding to DC cells induces the production of T_{H}2 cell attracting chemokines TARC (thymus and activation-regulated chemokine; also known as CCL17) and MDC (macrophage-derived chemokine, also known as CCL22), and upregulates costimulatory molecules HLA-DR, CD40, CD80, CD86, and CD83 on the surface of cells. TSLP-activated DCs in cell culture induced naïve CD4⁺ (Soumelis, *supra*) and CD8⁺ T cell differentiation into pro-allergic effector cells (Gilliet et al, J. Exp. Med. 197(8), 1059-1063 (2003)) which produce pro-allergic cytokines IL-4, IL-5, IL-13 and TNF-α while down-regulating IL-10 and interferon-y (Soumelis et al., *supra,* Gilliet et al., *supra*)*.* TSLP has been reported to be expressed in tissue samples of inflamed tonsilar epithelial cells, and keratinocytes within the lesions of atopic dermatis patients. (Soumelis et al., *supra*)*.*

### TSLP Assays

TSLP activities can be measured in an assay using BAF cells expressing human TSLPR (BAF/HTR), which require active TSLP for proliferation as described in PCT patent application publication WO 03/032898. The BAF/HTR bioassay utilizes a murine pro B lymphocyte cell line, which has been transfected with the human TSLP receptor (cell line obtained from Steven F. Ziegler, Virginia Mason Research Center, Seattle, WA.). The BAF/HTR cells are dependent upon huTSLP for growth, and proliferate in response to active huTSLP added in test samples. Following an incubation period, cell proliferation is measured by the addition of Alamar Blue dye I (Biosource International Catalog # DAL1100,10 uLwell). Metabolically active BAF/HRT cells take up and reduce Alamar Blue, which leads to change in the fluorescent properties of the dye. Additional assays for huTSLP activity include, for example, an assay measuring induction of T cell growth from human bone marrow by TSLP as described in U.S. Patent 6,555,520. Another TSLP activity is the ability to activate STAT5 as described in the reference to Levin et al., J. Immunol.162:677-683 (1999) and PCT patent application WO 03/032898. Additional assays include TSLP induced CCL17/TARC production from primary human monocytes and dendritic cells as described in the reference to Soumelis et al. *supra.*

TSLP has been found to induce fibroblast accumulation and collagen deposition in animals, as described in the Example below. Injection of murine TSLP intradermally into mice resulted in fibrosis within the subcutis of the mice, characterized by fibroblast proliferation and collagen deposition. Antagonizing TSLP activity would result in preventing or decreasing fibroblast proliferation and collagen deposition in a tissue.

As used herein the term "profibrotic factors" refers to cytokines, growth factors or chernokines in addition to TSLP which have been observed to promote the accumulation of fibroblasts and deposition of collagen in various tissues. A number of cytokines and growth factors have been reported to be involved in regulating tissue remodeling and fibrosis. These include the "profibrotic cytokines" such as transforming growth factor beta (TGF-ß), interleukin-4 (IL-4), interleukin-5 (IL-5), and interleukin-13 (IL-13), which have been shown to stimulate collagen synthesis and fibrosis in fibrotic tissues (Letterio et al. Ann Rev. Immunol. 16, 137-161 (1998), Fertin et al., Cell Mol. Biol. 37, 823-829 (1991), Doucet et al., J. Clin. Invest. 101, 2129-2139 (1998). Interleukin-9 (IL-9) has been shown to induce airway fibrosis in the lungs of mice

(Zhu et al., J. Clin. Invest. 103, 779-788(1999)). In addition to TGF-β, other cytokines or growth factors which have been reported to increase fibrosis in the fibrotic disorder idiopathic pulmonary fibrosis (IPF) include granulocyte/macrophage-colony stimulating factor (GM-CSF), tumor necrosis factor alpha (TNF-α), interleukin-1 beta (IL-1β), and connective tissue growth factor (CTGF) (Kelly et al. Curr Pharmaceutical Des 9: 39-49 (2003)). Cytokines and growth factors reported to be involved in promoting pulmonary fibrosis occurring in scleroderma include TGF-β, interleukin-1 beta (IL-1β), interleukin-6 (IL-6), oncostatin M (OSM), platelet derived growth factor (PDGF), the type 2 cytokines IL-4 and IL-13, IL-9, monocyte chemotactic protein 1 (CCL2/MCP-1), and pulmonary and activation- regulated chemokine (CCL18/PARC) (Atamas et al., Cyto Growth Fact Rev 14: 537-550 (2003)). Therefore, in one embodiment, the methods and compositions of the present invention further comprise administering at least one additional antagonist to one or more profibrotic factor in addition to at least one TSLP antagonist to reduce or prevent fibrosis in a subject suffering from a fibrotic disorder. In another aspect, the present invention provides for the use of at least one profibrotic antagonist in addition to at least one TSLP antagonist in the preparation of a medicament for the treatment or prevention of a fibrotic disorder in a subject. In another aspect, the present invention provides a pharmaceutical composition comprising, in addition to at least one TSLP antagonist, one or more antagonists to profibrotic factors, that is cytokines, growth factors or chemokines, in admixture with a pharmaceutically acceptable carrier. These profibrotic factors include, but are not limited to, the following cytokines, growth factors or chemokines: interleukin-4 (IL-4), interleukin-5 (IL-5), interleukin-9 (IL-9), interleukin-13 (IL-13), transforming growth factor beta (TGF-β), granulocyte/macrophage-colony stimulating factor (GM-CSF), tumor necrosis factor alpha (TNF-α), interleukin-1 beta (IL-1β), connective tissue growth factor (CTGF), interleukin-6 (IL-6), oncostatin M (OSM), platelet derived growth factor (PDGF), monocyte chemotactic protein 1 (CCL2/MCP-1), and pulmonary and activation- regulated chemokine (CCL18/PARC). The Accession numbers for these cytokines and their specific receptors (if available) are found in Table I below.

**TABLE I**

| Protein Name | Species | Synonyms | Database(s) (or Patent Application) | Accession No. (or SEQ ID No:) |
|---|---|---|---|---|
| TSLP | Homo sapiens | Thymic stromal lymphopoietin protein | GenBank/ US Patent No.6555520 | AAK67940/ SEQ ID NO: 2 |
| TSLP | Mus musculus | Thymic stroma derived lymphopoietin; Thymic stromal derived lymphopoietin | GenBank | AAF81677 |
| TSLPR | Homo sapiens | Cytokine receptor-like 2 (CRL2); IL-XR; Thymic stromal lymphopoietin protein receptor | US 2002/0068323 | SEQ ID NO: 5 |
| TSLPR | Mus | Cytokine receptor-like factor 2; Type I cytokine receptor delta 1; Cytokine receptor-like molecule 2 (CRLM-2); Thymic stromal lymphopoietin protein receptor | GenBank, SWISSPROT | Q8CII9 |
| TNF-alpha | Homo sapiens | Tumor necrosis factor; Tumor necrosis factor ligand superfamily member 2; TNF-a; Cachectin | GenBank, SWISSPROT | P01375 |
| TNF-alpha | Mus | Tumor necrosis factor; Tumor necrosis factor ligand superfamily member 2; TNF-a; Cachectin | GenBank, SWISSPROT | P06804 |
| TNF-RI | Homo sapiens | Tumor necrosis factor receptor superfamily member 1A; p60; TNF-R1; p55; CD120a [contains: Tumor necrosis factor binding protein 1 (TBPI)] | GenBank, SWISSPROT | P19438 |
| TNF-RI | Mus | Tumor necrosis factor receptor superfamily member 1A; p60; TNF-R1; p55 | GenBank, SWISSPROT | P25118 |
| TNF-RII | Homo sapiens | Tumor necrosis factor receptor superfamily member 1B; Tumor necrosis factor receptor 2; p80; TNF-R2; p75; CD120b; Etanercept [contains: Tumor necrosis factor binding protein 2 (TBPII)] | GenBank, SWISSPROT | P20333 |
| TNF-RII | Mus | Tumor necrosis factor receptor superfamily member 1B; Tumor necrosis factor receptor 2; TNF-R2; p75 | GenBank, SWISSPROT | P25119 |
| IL-1 alpha | Homo sapiens | Interleukin-1 alpha; Hematopoietin-1 | GenBank, SWISSPROT | P01583 |
| IL-1 alpha | Mus | Interleukin-1 alpha | GenBank, SWISSPROT | P01582 |
| IL-1 R-1 | Homo sapiens | Interleukin-1 receptor, type I; IL-1R-alpha; P80; Antigen CD121a | GenBank, SWISSPROT | P14778 |
| IL-1 R-1 | Mus | Interleukin-1 receptor, type I; P80 | GenBank, SWISSPROT | P13504 |
| IL-1 R-2 | Homo sapiens | Interleukin-1 receptor, type II; IL-1R-beta; Antigen CDw121b | GenBank, SWISSPROT | P27930 |
| IL-1 R-2 | Mus | Interleukin-1 receptor, type II | GenBank, SWISSPROT | P27931 |
| IL-4 | Homo sapiens | Interleukin-4; B-cell stimulatory factor 1 (BSF-1); Lymphocyte stimulatory factor 1 | GenBank, SWISSPROT | P05112 |
| IL-4 | Mus | Interleukin-4; B-cell stimulatory factor 1 (BSF-1); Lymphocyte stimulatory factor 1; IGG1 induction factor; B-cell IGG differentiation factor; B-cell growth factor 1 | GenBank, SWISSPROT | P07750 |
| IL-4R | Homo sapiens | Interleukin-4 receptor alpha chain (IL-4R-alpha; CD124 antigen) [contains: Soluble interleukin-4 receptor alpha chain (sIL4Ralpha/prot); IL-4-binding protein (IL4-BP)] | GenBank, SWISSPROT | P24394 |
| IL-4R | Mus | Interleukin-4 receptor alpha chain (IL-4R-alpha) [contains: Soluble interleukin-4 receptor alpha chain; IL-4-binding protein (IIA-BP)] | GenBank, SWISSPROT | P16382 |
| IL-5 | Homo sapiens | Interleukin-5; T-cell replacing factor (TRF); Eosinophil differentiation factor; B cell differentiation factor I | GenBank, SWISSPROT | P05113 |
| IL-5 | Mus | Interleukin-5; T-cell replacing factor (TRF); B-cell growth factor II (BCGF-II); Eosinophil differentiation factor; Cytotoxic T lymphocyte inducer | GenBank, SWISSPROT | P04401 |
| IL-5R | Homo sapiens | Interleukin-5 receptor alpha chain (IL-5R-alpha); CD125 antigen | GenBank, SWISSPROT | Q01344 |
| IL-5R | Mus | Interleukin-5 receptor alpha chain (IL-5R-alpha) | GenBank, SWISSPROT | P21183 |
| IL-9 | Homo sapiens | Interleukin-9; T-cell growth factor P40; P40 cytokine | GenBank, SWISSPROT | P15248 |
| IL-9 | Mus | Interleukin-9; T-cell growth factor P40; P40 cytokine | GenBank, SWISSPROT | P15247 |
| IL-9R | Homo sapiens | Interleukin-9 receptor | GenBank, SWISSPROT | Q01113 |
| IL-9R | Mus | Interleukin-9 receptor | GenBank, SWISSPROT | Q01114 |
| IL-13 | Homo sapiens | Interleukin-13 | GenBank, SWISSPROT | P35225 |
| IL-13 | Mus | Interleukin-13; T-cell activation protein P600 | GenBank, SWISSPROT | P20109 |
| IL-13RA-1 | Homo sapiens | Interleukin-13 receptor alpha-1 chain (IL-13R-alpha-1); CD213a1 antigen | GenBank, SWISSPROT | P78552 |
| IL-13RA-1 | Mus | Interleukin-13 receptor alpha-1 chain (IL-13R-alpha-1); Interleukin-13 binding protein; NR4 | GenBank, SWTSSPROT | 009030 |
| IL-13RA-2 | Homo sapiens | Interleukin-13 receptor alpha-2 chain; Interleukin-13 binding protein | GenBank, SWISSPROT | Q14627 |
| IL-13RA-2 | Mus musculus | IL-13 receptor alpha 2 | GenBank | AAC33240 |
| TGF-β1 | Homo sapiens | Transforming growth factor beta 1 | SWISSPROT | P01137 |
| TGF-β1 | Mus musculus | Transforming growth factor beta 1 | SWISSPROT | P04202 |
| TGF-β R1 | Homo sapiens | Transforming growth factor beta receptor type I ; serine/threonine-protein kinase receptor R4 (SKR4); activin receptor-like kinase 5 (ALK-5) | SWISSPROT | P36897 |
| TGF-β R2 | Homo sapiens | Transforming growth factor beta receptor type II | SWISSPROT | P37173 |
| GM-CSF | Homo sapiens | granulocyte-macrophage colony-stimulating factor; colony-stimulating factor; sargramostim; molgramostin | SWISSPROT | P04141 |
| IL-6 | Homo sapiens | Interleukin 6; interferon, beta 2 | Genbank | AAH15511 |
| IL-6 | Homo sapiens | Interleukin-6 precursor; B-cell stimulatory factor 2; interferon beta-2; hybridoma growth factor; CTL differentiation factor | SWISSPROT | P05231 |
| IL-6 | Mus musculus | Interleukin-6 precursor; interleukin HP-1; B-cell hybridoma growth factor | SWISSPROT | P08505 |
| IL-6 R β | Homo sapiens | interleukin-6 receptor beta chain; membrane glycoprotein 130; gp130; oncostatin M receptor; CDw130; CD130 antigen | SWISSPROT | P40189 |
| IL-6R-alpha | Homo sapiens | Interleukin-6 receptor alpha chain precursor; CD126 antigen | SWISSPROT | P08887 |
| IL-6R-beta | Homo sapiens | Interleukin-6 receptor beta chain | Genbank; SWISSPROT | AAB63010; |
| IL-6 R-alpha | Mus musculus | Interleukin-6 receptor alpha chain | SWISSPROT | P22272 |
| IL-6R-beta | Mus musculus | Intereleukin-6 receptor beta chain | SWISSPROT | Q00560 |
| OSM | Homo sapiens | Oncostatin M | Genbank | AAA36388 |
| OSMR-beta subunit | Homo sapiens | Oncostatin-M specific receptor beta subunit | Genbank; US Patent No. 5891997 | AAC50946; SEQ ID NO: 2 |
| OSM | Mus musculus | Oncostatin M precursor | SWISSPROT | P53347 |
| OSMR | Mus musculus | Oncostatin M specific receptor | Genbank | AAC40122 |
| CTGR | Homo sapiens | Connective tissue growth factor precursor; hypertrophic chondrocyte-specific protein 24 | SWISSPROT | P29279 |
| CTGR | Mus musculus | Connective tissue growth factor precursor; FISP-12 protein; hypertrophic chondrocyte-specific protein 24 | SWISSPROT | P29268 |
| PDGF-1 | Homo sapiens | Platelet-derived growth factor, A chain; platelet-derived growth factor alpha polypeptide | SWISSPROT | P04085 |
| PDGF-2 | Homo sapiens | Platelet-derived growth factor, B chain; platelet-derived growth factor beta polypeptide | SWISSPROT | P01127 |
| PDGF-1 | Mus musculus | Platelet-derived growth factor, A chain precursor | SWISSPROT P20033 | |
| PDGF-2 | Mus musculus | Platelet-derived growth factor, B chain precursor | SWISSPROT | P31240 |
| PDGR-R-α | Homo sapiens | Alpha platelet-derived growth factor receptor, CD140a antigen | SWISSPROT | P16234 |
| PDGR-R-β | Homo sapiens | Beta platelet-derived growth factor receptor; CD140B antigen | SWISSPROT | P09619 |
| CCL2 | Homo sapiens | Small inducible cytokine A2 precursor; monocyte chemotactic protein 1 (MCP-1); monocyte chemotactic and activating factor (MCAF); monocyte secretory protein JE (HC11) | SWISSPROT | P13500 |
| MCP-1-R | Homo sapiens | C-C chemokine receptor type 2 (CCR2); Monocyte chemoattractant protein 1 receptor | SWISSPROT | P41597 |
| CCL18 | Homo sapiens | Small inducible cytokine A18 precursor (CCL18); Macrophage inflammatory protein 4 (MIP-4); Pulmonary and activation-regulated chemokine (CC chemokine PARC); Alternative macrophage activation-associated CC chemokine 1 (MAC-1); Dendritic cell chemokine 1 (DC-CK1) | SWISSPROT | P55774 |

### TSLP antagonists

A TSLP antagonist inhibits or blocks at least one activity of TSLP, or alternatively, blocks expression of the cytokine or its receptor. Inhibiting or blocking cytokine activity can be achieved, for example, by employing one or more inhibitory agents which interfere with the binding of the cytokine to its receptor, and/or blocks signal transduction resulting from the binding of the cytokine to its receptor.

In the present invention, the TSLP antagonist comprises a TSLP binding agent, which binds to TSLP and prevents binding of the cytokine to its receptor, and/or blocks signal transduction resulting from the binding of the cytokine to its receptor. These antagonists include, but are not limited to, antagonistic antibodies, peptide or polypeptide binding agents, soluble TSLPR and soluble EL-7Rα/TSLPR heterodimers.

In another embodiment, the antagonist is a TSLPR antagonist, which binds to this receptor and blocks ligand binding and/or signal transduction. These antagonists include, but are not limited to, antagonistic antibodies and soluble ligands, which bind to TSLPR and interfere with TSLP signal transduction and activity.

In another embodiment, the antagonist is an antagonist to the IL-7Rα/TSLPR heterodimer, which binds to the heterodimer, and blocks ligand binding and/or signal transduction. These antagonists include, but are not limited to, antagonistic antibodies, soluble ligands, which bind to the heterodimer and interfere with TSLP signal transduction and activity.

In another embodiment, the uses and compositions of the present invention provide an additional antagonist to one or more "profibrotic factors", including but not limited to IL-4, IL- 5, IL-9, IL-13, TGF-β, GM-CSF, TNF-α, IL-1β, CTGF, IL-6, OSM, PDGF, CCL2/MCP-1, and CCL18/PARC to prevent or reduce fibrosis in a subject suffering from a fibrotic disorder. Antagonists to these profibrotic factors can be selected from agents which bind to the factor itself, the receptor, or a heterodimeric receptor to which the factor may bind and signal, wherein the antagonist interferes with ligand/receptor binding and/or at least one activity. In one embodiment, the factor antagonist blocks expression of the factor or its receptor.

In one embodiment, the TSLP antagonists specifically bind to the TSLP ligand, receptor or heterodimer receptor. As used herein the term "specifically binds" refers to antagonists such as antibodies have a binding affinity (Ka) for TSLP, TSLPR, or the heterodimer, (or corresponding to a profibrotic cytokine, cytokine receptor, or cytokine heterodimer receptor) of greater than or equal to 10⁶ M⁻¹, in one embodiment 10⁷ M⁻¹, in anther embodiment, 10⁸ M⁻¹, in another embodiment 10⁹ M⁻¹, as determined by techniques well known in the art (such as, for example Scatchard, Ann NY Acad Sci 51:660-672 (1949), and as described below).

The antagonists for TSLP and profibrotic factors generally are described in greater detail below.

### Particular Antagonists

### Antibodies

Antagonists include antibodies that bind to either a cytokine or its receptor and reduce or block at least one activity of the cytokine. As used herein, the term "antibody" refers to refers to intact antibodies including polyclonal antibodies (see, for example Antibodies: A Laboratory Manual, Harlow and Lane (eds), Cold Spring Harbor Press, (1988)), and monoclonal antibodies (see, for example, U.S. Patent Nos. RE 32,011, 4,902,614, 4,543,439, and 4,411,993, and Monoclonal Antibodies: A New Dimension in Biological Analysis. Plenum Press, Kennett, McKeam and Bechtol (eds.) (1980)). As used herein, the term "antibody" also refers to a fragment of an antibody such as F(ab), F(ab'), F(ab')₂, Fv, complementarity determining regions (CDR) fragments, single chain antibodies (scFv), or combinations of these, which can be produced by DNA recombinant techniques or by enzymatic or chemical cleavage of intact antibodies. Antibodies also include polypeptides such as fusion proteins that contain at least a portion of an immunogloblin that is sufficient to confer specific antigen binding to the polypeptide. Antibodies also include dAb (V_{H} domain), diabodies (bivalent antibodies comprising two polypeptide chains, each having V_{H} and V_{L} chains), and triabodies and tetrabodies (antibodies with three and four polypeptide chains respectively, each having V_{H} and V_{L} chains). Antibodies also include minibodies (as described in WO 94/09817), and maxibodies or scFv-Fc fusions (Powers et al, J. Immunol Meth 251, 123-135 (2001)), produced by recombinant DNA techniques or by enzymatic or chemical cleavage of intact antibodies.

The term "antibody" also refers to bispecific or bifunctional antibodies which are an artificial hybrid antibody having two different heavy/light chain pairs and two different binding sites. Bispecific antibodies can be produced by a variety of methods including fusion of hybridomas or linking of Fab' fragments. (See Songsivilai et al, Clin. Exp. Immunol. 79:315-321 (1990), Kostelny et al., J. Immunol. 148:1547-1553 (1992)). As used herein the term "antibody" also refers to chimeric antibodies, that is, antibodies having a human constant antibody immunoglobin domain is coupled to one or more non-human variable antibody immunoglobin domain, or fragments thereof (see, for example, U.S. Patent No. 5,595,898 and U.S. Patent No. 5,693,493). Antibodies also refer to "humanized" antibodies, and human antibodies produced by transgenic animals, both of which are described more fully below. The term "antibodies" also includes multimeric antibodies, or a higher order complex of proteins such as heterdimeric antibodies. "Antibodies" also includes anti-idiotypic antibodies. The production of antibodies is described in more detail below.

Polyclonal antibodies directed toward a cytokine or its receptor polypeptide may be produced in animals (e.g., rabbits or mice) by means of multiple subcutaneous or intraperitoneal injections of the polypeptide and an adjuvant. It may be useful to conjugate the antigen polypeptide to a carrier protein that is immunogenic in the species to be immunized, such as keyhole limpet hemocyanin, serum, albumin, bovine thyroglobulin, or soybean trypsin inhibitor. Also, aggregating agents such as alum may be used to enhance the immune response. After immunization, the animals are bled and the serum is assayed for antibody titer.

Monoclonal antibodies that are immunoreactive with a cytokine or its receptor are produced using any method that provides for the production of antibody molecules by continuous cell lines in culture. Examples of suitable methods for preparing monoclonal antibodies include the hybridoma methods of Kohler et al. Nature 256:495-97 (1975) and the human B-cell hybridoma method (Kozbor, J. Immunol. 133:3001 (1984); Brodeur et al., Monoclonal Antibody Production Techniques and Applications 51-63 (Marcel Dekker, Inc., 1987). Also provided by the invention are hybridoma cell lines that produce monoclonal antibodies reactive with cytokines or their receptors.

Monoclonal antibodies of the invention may be modified for use as therapeutics. One embodiment is a "chimeric" antibody in which a portion of the heavy (H) and/or light (L) chain is identical with or homologous to a corresponding sequence in antibodies derived from a particular species or belonging to a particular antibody class or subclass, while the remainder of the chain(s) is/are identical with or homologous to a corresponding sequence in antibodies derived from another species or belonging to another antibody class or subclass. Also included are fragments of such antibodies, so long as they exhibit the desired biological activity. See U.S. Patent No. 4,816,567; Morrison et al., Proc. Natl. Acad. Sci. 81:6851-55 (1985).

A monoclonal antibody may also be a "humanized" antibody. Methods for humanizing non-human antibodies are well known in the art. See U.S. Patent Nos. 5,585,089 and 5,693,762. Generally, a humanized antibody has one or more amino acid residues introduced into it from a source that is non-human. Humanization can be performed, for example, using methods described in the art (see, for example, U.S. Pat. No. 4,816,567 and WO 94/10332, Jones et al., Nature 321:522-25 (1986); Riechmann et al., Nature 332:323-27 (1998); Verhoeyen et al., Science 239:1534-36 (1988)), by substituting at least a portion of a rodent complementarity-determining region for the corresponding regions of a human antibody.

Antibodies may be human antibodies. Using transgenic animals (e.g., mice) that are capable of producing a repertoire of human antibodies in the absence of endogenous immunoglobulin production such antibodies are produced by immunization with the appropriate antigen (i.e., having at least 6 contiguous amino acids), optionally conjugated to a carrier. See, e.g., Jakobovits et al., Proc. Natl.Acad.Sci. 90:2551-55 (1993); Jakobovits et al., Nature 362:255-58 (1993) Bruggermann et al.Year in Immuno. 7:33 (1993), Mendez et al., Nature Genetics 15:146-156 (1997), and U.S. Patent No. 6,300,129,). In one method, such transgenic animals are produced by incapacitating the endogenous loci encoding the heavy and light immunoglobulin chains therein, and inserting loci encoding human heavy and light chain proteins into the genome thereof. Partially modified animals, that is those having less than the full complement of modifications, are then cross-bred to obtain an animal having all of the desired immune system modifications. When administered an immunogen, these transgenic animals produce antibodies with human (rather than, e.g., murine) amino acid sequences, including variable regions which are immunospecific for these antigens. See PCT Pub. Nos. WO9633735 and WO9402602 Additional methods are described in U.S. Patent No. 5,545,807, PCT Pub. Nos. WO9110741 and WO 9004036, and in European Patent Nos. 546073B1 and 546073A1. Human antibodies can also be produced by the expression of recombinant DNA in host cells or by expression in hybridoma cells as described herein.

Antibodies including human antibodies can also be produced from phage-display libraries (Hoogenboom et al., J. Mol. Biol. 227:381 (1991); Marks et al., J. Mol. Biol. 222:581(1991)). These processes mimic immune selection through the display of antibody repertoires on the surface of filamentous bacteriophage, and subsequent selection of phage by their binding to an antigen of choice. One such technique is described in WO 9910494 which describes the isolation of high affinity and functional agonistic antibodies for MPL- and msk-receptors using such an approach. Antibody phage display libraries are available in which Fab antibody fragments, for example, are displayed on phage and phagemid libraries, which allow for the selection and purification of soluble Fabs and IgGs, and which permit affinity purification (Dyax Corp).

Chimeric, CDR grafted, and humanized antibodies are typically produced by recombinant methods. Nucleic acids encoding the antibodies are introduced into host cells and expressed using materials and procedures described herein. In one embodiment, the antibodies are produced in mammalian host cells, such as CHO cells. Monoclonal (e.g., human) antibodies may be produced by the expression of recombinant DNA in host cells or by expression in hybridoma cells as described herein.

### Peptide/Polypeptide Antagonists

Antagonists to TSLP include peptides and polypeptides which are capable of binding to TSLP, TSLPR, or the IL-7Rα/TSLPR heterodimeric receptor, inhibiting or blocking ligandreceptor binding, and/or reducing or blocking cytokine activity. Peptide and polypeptide antagonists to other profibrotic factors include peptides or polypeptides capable of binding to the ligand, the ligand receptor, or a heterodimer receptor, where applicable. As used herein the term "polypeptide" refers to any chain of amino acids linked by peptide bonds, regardless of length or post-translational modification. "Peptide"generally refers to a shorter chain of amino acids, between approximately two amino acids to approximately fifty amino acids. Polypeptides and peptides include natural proteins, synthetic or recombinant polypeptides and peptides. As used herein, the term "amino acid" refers to the 20 standard α-amino acids as well as naturally occurring and synthetic derivatives. A polypeptide may contain L or D amino acids or a combination thereof. As used herein the term "peptidomimetic" refers to peptide-like structures which have non-amino acid structures substituted for one or more amino acids.

The binding polypeptides and peptides of the present invention can include a sequence or partial sequence of naturally occurring proteins, randomized sequences derived from naturally occurring proteins, or entirely randomized sequences.

Peptide and polypeptide antagonists include fusion proteins wherein the amino and/or carboxy termini of the peptide or polypeptide is fused to another polypeptide, a fragment thereof, or to amino acids which are not generally recognized to be part of any specific protein sequence. Examples of such fusion proteins are immunogenic polypeptides such as immunoglobulin constant regions (Fc), marker proteins, proteins or polypeptides that facilitate purification of the desired peptide or polypeptide, sequences that promote formation of multimeric proteins such as leucine zipper motifs that are useful in dimer or trimer formation and to promote stability and longer circulating half-lives. Other useful fusion proteins include the linking of functional domains, such as active sites from enzymes, glycosylation domains, cellular targeting signals or transmembrane regions. These peptides or polypeptides can be further attached to peptide linkers in addition to multimerizing agents such as an Fc region in order to multimerize the molecule and thereby enhance binding affinity. Fusions of antibody fragments such as the Fc domain of IgF, IgA, IgM, or IgE with a polypeptide such as a soluble domain of a cytokine receptor are well known. The binding peptides or polypeptides may also be attached to carrier molecules such as polyethylene glycol (PEG).

Binding polypeptides and peptides further include peptibodies, which are described in U.S. Patent No. 6,660,843.

### Soluble ligands

Peptide and polypeptide antagonists include soluble ligand antagonists. As used herein the term "soluble ligand antagonist" refers to soluble peptides, polypeptides or peptidomimetics capable of binding the TSLP receptor or other profibrotic factor receptor, or heterodimeric receptor and blocking cytokine-receptor binding and/or signal transduction and activity. Soluble ligand antagonists include variants of the cytokine which maintain substantial homology to, but not the activity of the ligand, including truncations such an N- or C-terminal truncations, substitutions, deletions, and other alterations in the amino acid sequence, such as substituting a non-amino acid peptidomimetic for an amino acid residue. Soluble ligand antagonists, for example, may be capable of binding the cytokine receptor, but not allowing signal transduction. For the purposes of the present invention a protein is "substantially similar" to another protein if they are at least 80%, preferably at least about 90%, more preferably at least about 95% identical to each other in amino acid sequence.

### Soluble receptors

Peptide and polypeptide antagonists further include truncated versions or fragments of the cytokine receptor, modified or otherwise, capable of binding to TSLP, (or other profibrotic factors) and/or blocking or inhibiting TSLP receptor binding, and thereby reducing or blocking cytokine activity. These truncated versions of the cytokine receptor, for example, includes naturally occurring soluble domains, as well as variations due to proteolysis of the N- or C-termini. The soluble domain includes all or part of the extracellular domain of the receptor, alone or attached to additional peptides or modifications. The soluble domain of human TSLPR is approximately amino acids 25 to 231 of SEQ ID NO: 4. The soluble domain of IL-7Rα is approximately amino acids 1 to 219 of Figure 2 of U.S. Patent No. 5,264,416. Soluble domains of receptors can also be provided as fusion proteins, such as Fc fusions.

Cytokine antagonists also include cross-linked homo or heterodimeric receptors or fragments of receptors designed to bind cytokines, also known as "cytokine traps". Cytokine traps are fusion polypeptides capable of binding a cytokine to form a non-functional complex. A cytokine trap includes at least a cytokine binding portion of an extracellular domain of the specificity determining region of a cytokine's receptor together with a cytokine binding portion of the extracellular domain of the signal transducing component of the cytokine's receptor and a component such as an Fc which multimerizes the cytokine receptor fragments. Cytokine traps are described, for example, in U.S. Patent No. 6,472,179.

### Peptides and polypeptides

The peptides and polypeptide antagonists of the present invention may be generated by any methods known in the art including chemical synthesis, digestion of proteins, or recombinant technology, phage display, RNA-peptide screening, and other affinity screening techniques. For example, polypeptides and peptides can be synthesized in solution or on a solid support in accordance with conventional techniques. Various automatic synthesizers are commercially available and can be used in accordance with known protocols. See, for example, Stewart and Young (supra); Tam et al., J Am Chem Soc, 105:6442, (1983); Merrifield, Science 232:341-347 (1986); Barany and Merrifield, The Peptides, Gross and Meienhofer, eds, Academic Press, New York, 1-284; Barany et al., Int JPep Protein Res, 30:705-739 (1987); and U.S. Patent No. 5,424,398. Methods for solid phase peptide synthesis are described in Coligan et al., Curr Prot Immunol, Wiley Interscience, 1991, Unit 9, for example.

Solid phase peptide synthesis methods use a copoly(styrene-divinylbenzene) containing 0.1-1.0 mM amines/g polymer. These methods for peptide synthesis use butyloxycarbonyl (t-BOC) or 9-fluorenylmethyloxy-carbonyl(FMOC) protection of alpha-amino groups. Both methods involve stepwise syntheses whereby a single amino acid is added at each step starting from the C-terminus of the peptide (See, Coligan et al., Curr Prot Immunol, Wiley Interscience, 1991, Unit 9). On completion of chemical synthesis, the synthetic peptide can be deprotected to remove the t-BOC or FMOC amino acid blocking groups and cleaved from the polymer by treatment with acid at reduced temperature (e.g., liquid HF-10% anisole for about 0.25 to about 1 hours at 0°C). After evaporation of the reagents, the peptides are extracted from the polymer with 1% acetic acid solution that is then lyophilized to yield the crude material. This can normally be purified by such techniques as gel filtration on Sephadex G-15 using 5% acetic acid as a solvent. Lyophilization of appropriate fractions of the column will yield the homogeneous peptides or peptide derivatives, which can then be characterized by such standard techniques as amino acid analysis, thin layer chromatography, high performance liquid chromatography, ultraviolet absorption spectroscopy, molar rotation, solubility, and quantitated by the solid phase Edman degradation.

Phage display techniques represent another method for identifying peptides capable of binding the cytokines or their receptors. Briefly, a phage library is prepared (using e.g. ml 13, fd, or lambda phage), displaying inserts of amino acid residues. The inserts may represent, for example, a completely degenerate or biased array. Phage-bearing inserts that bind to the desired antigen are selected and this process repeated through several cycles of reselection of phage that bind to the desired antigen. DNA sequencing is conducted to identify the sequences of the expressed peptides. The minimal linear portion of the sequence that binds to the desired antigen can be determined in this way. The procedure can be repeated using a biased library containing inserts containing part or all of the minimal linear portion plus one or more additional degenerate residues upstream or downstream thereof. These techniques may identify peptides with still greater binding affinity for the cytokines or their receptors. Phage display technology is described, for example, in Scott et al. Science 249: 386 (1990); Devlin et al., Science 249: 404 (1990); U.S. Patent No. 5,223,409; U.S. Patent No. 5,733,731; U.S. Patent No. 5,498,530; U.S. Patent No. 5,432,018; U.S. Patent No. 5,338,665; U.S. Patent No. 5,922,545; WO 96/40987, and WO 98/15833. Optionally, mutagenesis libraries are created and screened as described above to further optimize the sequence of the best binders.(Lowman, Ann Rev Biophys Biomol Struct 26:401-24 (1997)).

Other methods of generating binding peptides include additional affinity selection techniques known in the art, including *"E. coli* display", "ribosome display" methods employing chemical linkage of peptides to RNA known collectively as "RNA-peptide screening." Yeast two-hybrid screening methods also may be used to identify peptides of the invention that bind to cytokines or their receptors. In addition, chemically derived peptide libraries have been developed in which peptides are immobilized on stable, non-biological materials, such as olyethylene rods or solvent-permeable resins. Another chemically derived peptide library uses photolithography to scan peptides immobilized on glass slides. Hereinafter, these and related methods are collectively referred to as "chemical-peptide screening." Chemical peptide screening may be advantageous in that it allows use of D-amino acids and other analogues, as well as non-peptide elements. Both biological and chemical methods are reviewed in Wells and Lowman, Curr Opin Biotechnol 3: 355-62 (1992).

Additionally, selected peptides, peptidomimetics, capable of binding cytokines and cytokine receptors can be further improved through the use of "rational drug design". In one approach, the three-dimensional structure of a polypeptide of the invention, a ligand or binding partner, or of a polypeptide-binding partner complex, is determined by x-ray crystallography, by nuclear magnetic resonance, or by computer homology modeling or, most typically, by a combination of these approaches. Relevant structural information is used to design analogous molecules, to identify efficient inhibitors, such as small molecules that may bind to a polypeptide of the invention. Examples of algorithms, software, and methods for modeling substrates or binding agents based upon the three-dimensional structure of a protein are described in PCT publication WO107579.

Antagonists such as peptides, polypeptides, peptidometics, antibodies, soluble domains, are selected by screening for binding to the target cytokine or cytokine receptor targets, followed by non-specific and specific elution. A number of binding assays are known in the art and include non-competitive and competitive binding assays. Subsequently inhibitory parameters such as IC₅₀ (concentration at which 50% of a designated activity is inhibited) and the binding affinity as measured by K_{D} (dissociation constant) or Ka (association constant) can be determined using cell-based or other assays. IC₅₀ can be determined used cell based assays, for example, employing cell cultures expressing cytokine receptors on the cell surface, as well as a cytokine-responsive signaling reporter such as a pLuc-MCS reporter vector (Stratagene cat # 219087). The inhibition of signaling when increasing quantities of inhibitor is present in the cell culture along with the cytokine can be used to determine IC₅₀. As used herein, the term "specifically binds" refers to a binding affinity of at least 10⁶ M⁻¹, in one embodiment, 10⁷ M⁻¹ or greater. Equilibrium constant K_{D} or Ka can be determined by using BIAcore® assay systems such as BIAcore®3000 (Biacore, Inc., Piscataway, NJ) using various concentrations of candidate inhibitors according to the manufacturer's suggested protocol. The therapeutic value of the antagonists can then be tested on various animal models such as the murine models described below in the Example.

Specific antagonists to profibrotic factors are known. In addition to inhibitors to TSLP activity, the methods and compositions of the present invention can employ specific antagonists including the TNF-α receptor Fc fusion protein known as etanercept (ENBREL®), sTNF-RI, onercept, D2E7, and Remicade™, and antibodies specifically reactive with TNF-α and TNF-α receptor. Antagonists further include IL-1rα antagonist molecules such as anakinra, Kineret®, IL-1rα-like molecules such as IL-1Hy1 and Il-1Hy2; polypeptide inhibitors to IL-1α and IL-1α receptor, IL-1 soluble receptor antagonist. IL-1 polypeptide inhibitors are described in U.S. Patent 6,599,873, describing glycosylated and nonglycosylated polypeptide sequences, which is herein incorporated by reference. Kineret® differs from native human IL-1rα in that it has the addition of a single methionine residue at its amino terminus. Kineret^{®} blocks the biologic activity of IL-1 by competitively inhibiting IL-1 binding to the interleukin-1 type I receptor (IL-1rI). Additional known inhibitors include antibodies which bind to IL-4 and IL-4 receptor, antibodies which bind to IL-5 and IL-5 receptors, and antibodies which bind to IL-13 and IL-13 receptors.

Regardless of the manner in which the peptides or polypeptides are prepared, a nucleic acid molecule encoding each peptide or polypeptide can be generated using standard recombinant DNA procedures. The nucleotide sequence of such molecules can be manipulated as appropriate without changing the amino acid sequence they encode to account for the degeneracy of the nucleic acid code as well as to account for codon preference in particular host cells. Recombinant DNA techniques also provide a convenient method for preparing polypeptide agents of the present invention, or fragments thereof including soluble receptor domains, for example. A polynucleotide encoding the polypeptide or fragment may be inserted into an expression vector, which can in turn be inserted into a host cell for production of the polypeptides of the present invention.

A variety of expression vector/host systems may be utilized to express the peptides and polypeptide agents. These systems include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells.

The term "expression vector" refers to a plasmid, phage, virus or vector, for expressing a polypeptide from a polynucleotide sequence. An expression vector can comprise a transcriptional unit comprising an assembly of (1) a genetic element or elements having a regulatory role in gene expression, for example, promoters or enhancers, (2) a structural or sequence that encodes the polypeptide agent which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an amino terminal methionyl residue. This residue may or may not be subsequently cleaved from the expressed recombinant protein to provide a final polypeptide product. For example, the peptides and peptibodies may be recombinantly expressed in yeast using a commercially available expression system, e.g., the Pichia Expression System (Invitrogen, San Diego, CA), following the manufacturer's instructions. This system also relies on the pre-pro-alpha sequence to direct secretion, but transcription of the insert is driven by the alcohol oxidase (AOX1) promoter upon induction by methanol. The secreted polypeptide is purified from the yeast growth medium using the methods used to purify the polypeptide from bacterial and mammalian cell supernatants.

Alternatively, the cDNA encoding the peptide and polypeptides can be cloned into the baculovirus expression vector pVL1393 (PharMingen, San Diego, CA). This vector can be used according to the manufacturer's directions (PharMingen) to infect Spodoptera frugiperda cells in sF9 protein-free media and to produce recombinant protein. The recombinant protein can be purified and concentrated from the media using a heparin-Sepharose column (Pharmacia).

Alternatively, the peptide or polypeptide may be expressed in an insect system. Insect systems for protein expression are well known to those of skill in the art. In one such system, Autographa californica nuclear polyhedrosis virus (AcNPV) can be used as a vector to express foreign genes in Spodoptera frugiperda cells or in Trichoplusia larvae. The peptide coding sequence can be cloned into a nonessential region of the virus, such as the polyhedrin gene, and placed under control of the polyhedrin promoter. Successful insertion of the peptide will render the polyhedrin gene inactive and produce recombinant virus lacking coat protein. The recombinant viruses can be used to infect S. frugiperda cells or Trichoplusia larvae in which the peptide is expressed (Smith et al., J Virol 46: 584 (1983); Engelhard et al., Proc Nat Acad Sci (USA) 91: 3224-7 (1994)).

In another example, the DNA sequence encoding the peptide can be amplified by PCR and cloned into an appropriate vector for example, pGEX-3X (Pharmacia). The pGEX vector is designed to produce a fusion protein comprising glutathione-S-transferase (GST), encoded by the vector, and a protein encoded by a DNA fragment inserted into the vector's cloning site. The primers for PCR can be generated to include for example, an appropriate cleavage site.

Alternatively, a DNA sequence encoding the peptide can be cloned into a plasmid containing a desired promoter and, optionally, a leader sequence (Better et al., Science 240:1041-43 (1988)). The sequence of this construct can be confirmed by automated sequencing. The plasmid can then be transformed into E. coli strain MC1061 using standard procedures employing CaCl₂ incubation and heat shock treatment of the bacteria (Sambrook et al., supra). The transformed bacteria can be grown in LB medium supplemented with carbenicillin, and production of the expressed protein can be induced by growth in a suitable medium. If present, the leader sequence can effect secretion of the peptide and be cleaved during secretion.

Mammalian host systems for the expression of recombinant peptides and polypeptides are well known to those of skill in the art. Host cell strains can be chosen for a particular ability to process the expressed protein or produce certain post-translation modifications that will be useful in providing protein activity. Such modifications of the protein include, but are not limited to, acetylation, carboxylation, glycosylation, phosphorylation, lipidation and acylation. Different host cells such as CHO, HeLa, MDCK, 293, WI38, and the like have specific cellular machinery and characteristic mechanisms for such post-translational activities and can be chosen to ensure the correct modification and processing of the introduced, foreign protein.

It is preferable that transformed cells be used for long-term, high-yield protein production. Once such cells are transformed with vectors that contain selectable markers as well as the desired expression cassette, the cells can be allowed to grow for 1-2 days in an enriched media before they are switched to selective media. The selectable marker is designed to allow growth and recovery of cells that successfully express the introduced sequences. Resistant clumps of stably transformed cells can be proliferated using tissue culture techniques appropriate to the cell line employed.

A number of selection systems can be used to recover the cells that have been transformed for recombinant protein production. Such selection systems include, but are not limited to, HSV thymidine kinase, hypoxanthine-guanine phosphoribosyltransferase and adenine phosphoribosyltransferase genes, in tk-, hgprt- or aprt- cells, respectively. Also, anti-metabolite resistance can be used as the basis of selection for dhfr which confers resistance to methotrexate; gpt which confers resistance to mycophenolic acid; neo which confers resistance to the aminoglycoside G418 and confers resistance to chlorsulfuron; and hygro which confers resistance to hygromycin. Additional selectable genes that may be useful include trpB, which allows cells to utilize indole in place of tryptophan, or hisD, which allows cells to utilize histinol in place of histidine. Markers that give a visual indication for identification of transformants include anthocyanins, ß-glucuronidase and its substrate, GUS, and luciferase and its substrate, luciferin.

In some cases, the expressed polypeptides of this invention may need to be "refolded" and oxidized into a proper tertiary structure and disulfide linkages generated in order to be biologically active. Refolding can be accomplished using a number of procedures well known in the art. Such methods include, for example, exposing the solubilized polypeptide agent to a pH usually above 7 in the presence of a chaotropic agent. The selection of chaotrope is similar to the choices used for inclusion body solubilization, however a chaotrope is typically used at a lower concentration. Exemplary chaotropic agents are guanidine and urea. In most cases, the refolding/oxidation solution will also contain a reducing agent plus its oxidized form in a specific ratio to generate a particular redox potential which allows for disulfide shuffling to occur for the formation of cysteine bridges. Some commonly used redox couples include cysteine/cystamine, glutathione/dithiobisGSH, cupric chloride, dithiothreitol DTT/dithiane DTT, and 2-mercaptoethanol (bME)/dithio-bME. In many instances, a co-solvent may be used to increase the efficiency of the refolding. Commonly used cosolvents include glycerol, polyethylene glycol of various molecular weights, and arginine.

It is necessary to purify the peptides and polypeptides of the present invention. Protein purification techniques are well known to those of skill in the art. These techniques involve, at one level, the crude fractionation of the proteinaceous and non-proteinaceous fractions. Having separated the peptides or polypeptides from other proteins, the peptide or polypeptide of interest can be further purified using chromatographic and electrophoretic techniques to achieve partial or complete purification (or purification to homogeneity). Analytical methods particularly suited to the preparation of polypeptides and peptides are ion-exchange chromatography, exclusion chromatography; polyacrylamide gel electrophoresis; isoelectric focusing. A particularly efficient method of purifying peptides is fast protein liquid chromatography or even HPLC. The term "purified polypeptide or peptide" as used herein, is intended to refer to a composition, isolatable from other components, wherein the polypeptide or peptide is purified to any degree relative to its naturally-obtainable state. A purified peptide or polypeptide therefore also refers to a polypeptide or peptide that is free from the environment in which it may naturally occur. Generally, "purified" will refer to a peptide or polypeptide composition that has been subjected to fractionation to remove various other components, and which composition substantially retains its expressed biological activity. Where the term "substantially purified" is used, this designation will refer to a peptide or polypeptide composition in which the polypeptide or peptide forms the major component of the composition, such as constituting about 50%, about 60%, about 70%, about 80%, about 90%, about 95% or more of the proteins in the composition.

Various methods for quantifying the degree of purification of the peptide or polypeptide will be known to those of skill in the art in light of the present disclosure. These include, for example, determining the specific binding activity of an active fraction, or assessing the amount of peptide or polypeptide within a fraction by SDS/PAGE analysis. A preferred method for assessing the purity of a peptide or polypeptide fraction is to calculate the binding activity of the fraction, to compare it to the binding activity of the initial extract, and to thus calculate the degree of purification, herein assessed by a "-fold purification number." The actual units used to represent the amount of binding activity will, of course, be dependent upon the particular assay technique chosen to follow the purification and whether or not the polypeptide or peptide exhibits a detectable binding activity.

Various techniques suitable for use in purification will be well known to those of skill in the art. These include, for example, precipitation with ammonium sulphate, PEG, antibodies (immunoprecipitation) and the like or by heat denaturation, followed by centrifugation; chromatography steps such as affinity chromatography (e.g., Protein-A-Sepharose), ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be ornitted, and still result in a suitable method for the preparation of a substantially purified polypeptide.

### Antagonists to polynucleotides

Antagonists to TSLP and other profibrotic cytokines include antagonists which prevent or reduce expression of the cytokine or its receptor. These include antisense or sense oligonucleotides comprising a single-stranded polynucleotide sequence (either RNA or DNA) capable of binding to target mRNA (sense) or DNA (antisense) sequences. Antisense or sense oligonucleotides, comprise fragments of the targeted polynucleotide sequence encoding either the cytokine or its receptor. Such a fragment generally comprises at least about 14 nucleotides, typically from about 14 to about 30 nucleotides. The ability to derive an antisense or a sense oligonucleotide, based upon a nucleic acid sequence encoding a given protein is described in, for example, Stein and Cohen (Cancer Res. 48:2659, 1988), and van der Krol et al. (BioTechniques 6:958,1988). Binding of antisense or sense oligonucleotides to target nucleic acid sequences results in the formation of duplexes that block or inhibit protein expression by one of several means, including enhanced degradation of the mRNA by RNAse H, inhibition of splicing, premature termination of transcription or translation, or by other means. The antisense oligonucleotides thus may be used to block expression of proteins. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable in vivo (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly- (L)-lysine. Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid by any gene transfer method, including, for example, lipofection, CaPO₄-mediated DNA transfection, electroporation, or by using gene transfer vectors such as Epstein-Barr virus or adenovirus.

Sense or antisense oligonucleotides also may be introduced into a cell containing the target nucleic acid by formation of a conjugate with a ligand-binding molecule, as described in WO 91/04753. Suitable ligand binding molecules include, but are not limited to, cell surface receptors, growth factors, other cytokines, or other ligands that bind to cell surface receptors. Preferably, conjugation of the ligand-binding molecule does not substantially interfere with the ability of the ligand-binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid by formation of an oligonucleotide-lipid complex, as described in WO 90/10448. The sense or antisense oligonucleotide-lipid complex is preferably dissociated within the cell by an endogenous lipase.

Additional methods for preventing expression of targeted cytokines or cytokine receptors is RNA interference or RNA produced by the introduction of specific double-stranded RNA (dsRNA), as described, for example in Bosher et al., Nature Cell Biol 2, E31-E36 (2000).

### Pharmaceutical Compositions

Pharmaceutical compositions containing one or more TSLP antagonists according to the present invention are within the scope of the present invention. In addition pharmaceutical compositions containing one or more TSLP antagonists in addition to antagonists to profibrotic factors are provided. Such compositions comprise a therapeutically or prophylactically effective amount of each antagonist in admixture with pharmaceutically acceptable materials. An effective amount, as used herein, is an amount sufficient to treat a subject for a fibrotic disorder. Typically, the antagonists will be sufficiently purified for administration to an animal.

The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates, other organic acids); bulking agents (such as mannitol or glycine), chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride, mannitol sorbitol); delivery vehicles; diluents; excipients and/or pharmaceutical adjuvants. (Remington's Pharmaceutical Sciences, 18th Edition, A.R. Gennaro, ed., Mack Publishing Company, 1990).

The optimal pharmaceutical composition will be determined by one skilled in the art depending upon, for example, the intended route of administration, delivery format, and desired dosage. See for example, Remington's Pharmaceutical Sciences, *supra.* Such compositions may influence the physical state, stability, rate of in vivo release, and rate of in vivo clearance of the therapeutic molecule.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute therefore. In one embodiment of the present invention, antagonist compositions may be prepared for storage by mixing the selected composition having the desired degree of purity with optional formulation agents (Remington's Pharmaceutical Sciences, supra) in the form of a lyophilized cake or an aqueous solution. Further, the therapeutic antagonist may be formulated as a lyophilizate using appropriate excipients such as sucrose.

The pharmaceutical compositions can be selected for the condition to be treated. Treatment of fibrotic disorders may be delivered topically, orally or delivered by injection, for example. Alternatively, the compositions may be delivered, for example, by inhalation therapy, orally, or by injection. The preparation of such pharmaceutically acceptable compositions is within the skill of the art.

The formulation components are present in concentrations that are acceptable to the site of administration. For example, buffers are used to maintain the composition at physiological pH or at slightly lower pH, typically within a pH range of from about 5 to about 8.

When parenteral administration is contemplated, the therapeutic compositions for use in this invention may be in the form of a pyrogen-free, parenterally acceptable aqueous solution comprising the desired antagonist in a pharmaceutically acceptable vehicle. A particularly suitable vehicle for parenteral injection is sterile distilled water in which an antagonist is formulated as a sterile, isotonic solution, properly preserved. Yet another preparation can involve the formulation of the desired molecule with an agent, such as injectable microspheres, bio-erodible particles, polymeric compounds (polylactic acid, polyglycolic acid), beads, or liposomes, that provides for the controlled or sustained release of the product which may then be delivered via a depot injection. Hyaluronic acid may also be used, and this may have the effect of promoting sustained duration in the circulation. Other suitable means for the introduction of the desired molecule include implantable drug delivery devices.

In another aspect, pharmaceutical formulations suitable for parenteral administration may be formulated in aqueous solutions, preferably in physiologically compatible buffers such as Hanks' solution, ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions. In another embodiment, a pharmaceutical composition may be formulated for inhalation. For example, an antagonist may be formulated as a dry powder for inhalation. Antagonists including polypeptide or nucleic acid molecule inhalation solutions may also be formulated with a propellant for aerosol delivery. In yet another embodiment, solutions may be nebulized. Pulmonary administration is further described in PCT Application No. PCT/US94/001875, which describes pulmonary delivery of chemically modified proteins.

It is also contemplated that certain formulations may be administered orally. In one embodiment of the present invention, molecules that are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the antagonist molecule. Diluents, flavorings, low melting point waxes, vegetable oils, lubricants, suspending agents, tablet disintegrating agents, and binders may also be employed.

Pharmaceutical compositions for oral administration can also be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combining active compounds with solid excipient and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol, and sorbitol; starch from corn, wheat, rice, potato, or other plants; cellulose, such as methyl cellulose, hydroxypropylmethyl-cellulose, or sodium carboxymethylcellulose; gums, including arabic and tragacanth; and proteins, such as gelatin and collagen. If desired, disintegrating or solubilizing agents may be added, such as the cross-linked polyvinyl pyrrolidone, agar, and alginic acid or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations that can be used orally also include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Another pharmaceutical composition may involve an effective quantity of antagonist in a mixture with non-toxic excipients that are suitable for the manufacture of tablets. By dissolving the tablets in sterile water, or other appropriate vehicle, solutions can be prepared in unit dose form. Suitable excipients include, but are not limited to, inert diluents, such as calcium carbonate, sodium carbonate or bicarbonate, lactose, or calcium phosphate; or binders, such as starch, gelatin, or acacia; or lubricating agents such as magnesium stearate, stearic acid, or talc.

Additional pharmaceutical compositions will be evident to those skilled in the art, including formulations involving molecules in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, WO 9315722 that describes controlled release of porous polymeric microparticles for the delivery of pharmaceutical compositions. Additional examples of sustained-release preparations include semipermeable polymer matrices in the form of shaped articles, e.g. films, or microcapsules. Sustained release matrices may include polyesters, hydrogels, polylactides (U.S. 3,773,919, EP 58,481), copolymers of L-glutamic acid and gamma ethyl-L-glutamate (Sidman et al., Biopolymers, 22:547-556 (1983), poly (2-hydroxyethyl-methacrylate) (Langer et al., J. Biomed. Mater. Res., 15:167-277, (1981); Langer et al., Chem. Tech., 12:98-105(1982)), ethylene vinyl acetate (Langer et al., supra) or poly-D(-)-3-hydroxybutyric acid (EP 133,988). Sustained-release compositions also include liposomes, which can be prepared by any of several methods known in the art. See e.g., Eppstein et al.,PNAS (USA), 82:3688 (1985); EP 36,676; EP 88,046; EP 143,949.

The pharmaceutical composition to be used for in vivo administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

Kits for producing a single- dose administration unit may each contain both a first container having a dried protein and a second container having an aqueous formulation. Kits may contain single and multi-chambered pre-filled syringes (e.g., liquid syringes and lyosyringes).

An effective amount of a pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the molecule is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.1mg/kg to up to about 100 mg/kg or more, depending on the factors mentioned above. Antibodies may be preferably injected or administered intravenously.

For any compound, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans.

The exact dosage will be determined in light of factors related to the subject requiring treatment. Dosage and administration are adjusted to provide sufficient levels of the active compound or to maintain the desired effect. Factors that may be taken into account include the severity of the inflammatory condition, whether the condition is acute or chronic, the general health of the subject, the age, weight, and gender of the subject, time and frequency of administration, drug combination(s), reaction sensitivities, and response to therapy. Long-acting pharmaceutical compositions may be administered every 3 to 4 days, every week, or biweekly depending on the half-life and clearance rate of the particular formulation.

The frequency of dosing will depend upon the pharmacokinetic parameters of the therapeutic antagonist molecule in the formulation used. Typically, a composition is administered until a dosage is reached that achieves the desired effect. The composition may therefore be administered as a single dose, or as multiple doses (at the same or different concentrations/dosages) over time, or as a continuous infusion. Further refinement of the appropriate dosage is routinely made. Appropriate dosages may be ascertained through use of appropriate dose-response data. In addition, the composition may be administered prophylactically.

The route of administration of the pharmaceutical composition is in accord with know methods, e.g. orally, through injection by intravenous, intraperitoneal, intracerebral (intraparenchymal), intracerebroventricular, intramuscular, intra-ocular, intraarterial, intraportal, intralesional routes, intramedullary, intrathecal, intraventricular, transdermal, subcutaneous, intraperitoneal, intranasal, enteral, topical, sublingual, urethral, vaginal, or rectal means, by sustained release systems or by implantation devices. Where desired, the compositions may be administered by bolus injection or continuously by infusion, or by implantation device.

Alternatively or additionally, the composition may be administered locally via implantation of a membrane, sponge, or another appropriate material on to which the desired molecule has been absorbed or encapsulated. Where an implantation device is used, the device may be implanted into any suitable tissue or organ, and delivery of the desired molecule may be via diffusion, timed-release bolus, or continuous administration.

In some cases, an antagonist of the present invention can be delivered by implanting certain cells that have been genetically engineered, using methods such as those described herein, to express and secrete the polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semipermeable polymeric enclosures or membranes that allow the release of the protein product(s) but prevent the destruction of the cells by the patient's immune system or by other detrimental factors from the surrounding tissues.

Pharmaceutical compositions containing the therapeutic antagonists of the present invention are administered to a subject suffering from a fibrotic disorder to prevent or reduce fibrosis in the subject. Fibrotic disorders include local and systemic scleroderma, interstitial lung disease, idiopathic pulmonary fiborisis, fibrosis arising from chronic hepatitis B or C, radiation-induced fibrosis, and fibrosis arising from wound healing.

The invention having been described, the following examples are offered by way of illustration, and not limitation.

### EXAMPLE

Murine TSLP (R&D Systems) was administered to 15 8 week old Balb/c female mice (Charles River) according to the following protocol. The mice were divided into three groups of 5 mice each. Group 1 was injected three times a week for one week (three injections total); Group 2 was injected three times a week for two weeks (six injections total), and Group 3 was injected three times a week for six weeks (18 injections total). The mice were injected intradermally with 10 ug of TSLP in 100 ul of PBS on the left flank, and 100 ul of PBS on the right flank as a control. 72 hours after the final injection, the animals were anesthetized, terminal bleeds performed, and the serum isolated for future analysis. The skin was harvested, fixed in formalin, and made into slides for H&E (Hematoxylin and Eosin) staining for pathological evaluation.

Histopathological examination determined that after one or two weeks of intradermal muTSLP injections, the skin of mice contained infiltrates of mononuclear cells and eosinophils within the subcutis, with extension into the cutaneous trunci muscle and overlying adipose. The sites treated with TSLP also showed mild to moderate edema and minimal to moderate epithelial hyperplasia in the skin. In contrast, sections of skin injected with PBS showed only minimal mononuclear cell and eosinophil infiltrates along the injection sites. The skin lesions tended to be multifocal to locally extensive. Lesion severity increased with increasing duration of treatment. However, after 6 weeks of injection, the TSLP injected skin showed no signs of developing flakiness or lesions.

The skin sections were stained with Masson's trichrome, which stains the collagen green. Staining showed that at the two week time point collagen was starting to be deposited in TSLP treated vs. PBS treated skin. This collagen deposit was not seen at the one-week time point, but showed up at the two week and six week time points.

Histopathology showed that after six weeks of intradermal administration of muTSLP, the subcutis contained diffuse moderate to severe infiltrates of mononuclear cells and eosinophils; the dermis contained mast cells, eosinophils and mononuclear cells, and the epithelium was mildly hyperplastic. Skin injected with PBS showed only diffuse infiltrates of mononuclear cells and eosinophils within the dermis, possibly caused by systemic muTSLP or a non-specific reaction to repeated injections.

Six weeks of TSLP treatment resulted in moderate fibrosis within the subcutis, characterized by fibroblast proliferation and collagen deposition. This observation was confirmed with Trichrome staining. Neither fibroblast proliferation or collagen deposition was seen in the PBS treated subcutis. Staining of samples taken at six weeks showed an increased number of mast cells with in the dermis of inflamed muTSLP injected skin relative to a sparse population of mast cells in the dermis of PBS-tested skin.

The pathology scores for TSLP treated mouse skin at 1 week, 2 weeks, and six weeks after 3 injections per week compared with the PBS treated mouse skin at six weeks are summarized in Tables 2 to 5 shown below.

**Table 2: 1 week TSLP treatment**

| Treatment Animal No. | Group 1 | | | | | | |
|---|---|---|---|---|---|---|---|
| | TSLP wk1 | | | | | | |
| | 1-1 | 1-2† | 1-3† | 1-4† | 1-5 | Mean | SE |
| Inflammation | 0 | 3 | 1 | 2 | 0 | 1.2 | 0.6 |
| Neutrophils | 0 | 1 | 1 | 1 | 0 | 0.6 | 0.2 |
| Mononuclear cells | 0 | 3 | 1 | 2 | 0 | 1.2 | 0.6 |
| Eosinophils | 0 | 3 | 1 | 2 | 0 | 1.2 | 0.6 |
| Edema | 0 | 2 | 2 | 2 | 0 | 1.2 | 0.5 |
| Epithelial hyperplasia | | 0 2 | 1 | 1 | 0 | 0.8 | 0.4 |
| *Total | 0 | 7 | 4 | 5 | 0 | | |
| *Mean Group Score | | | | | | 3.2 | 1.4 |

**Table 3: 2 week TSLP treatment**

| Treatment Animal No. | Group 2 | | | | | | |
|---|---|---|---|---|---|---|---|
| | TSLP wk2 | | | | | | |
| | 2-1 | 2-2† | 2-3† | 2-4† | 2-5 | Mean | SE |
| Inflammation | 3 | 2 | 2 | 3 | 4 | 2.8 | 0.4 |
| Neutrophils | 1 | 1 | 1 | 1 | 1 | 1.0 | 0.0 |
| Mononuclear cells | 3 | 2 | 2 | 3 | 4 | 2.8 | 0.4 |
| Eosinophils | 3 | 2 | 2 | 3 | 4 | 2.8 | 0.4 |
| Edema | 2 | 2 | 2 | 2 | 3 | 2.2 | 0.2 |
| Epithelial hyperplasia | 1 | 1 | 2 | 2 | 2 | 1.6 | 0.2 |
| *Total | 6 | 5 | 6 | 7 | 9 | | |
| *Mean Group Score | | | | | | 6.6 | 0.7 |

**Table 4: 6 week TSLP treatment**

| Treatment Animal No. | Group 3A | | | | | | |
|---|---|---|---|---|---|---|---|
| | TSLP wk6 | | | | | | |
| | 3-1 | 3-2† | 3-3† | 3-4† | 3-5 | Mean | SE |
| Inflammation | 3 | 3 | 4 | 4 | 3 | 3.4 | 0.2 |
| Neutrophils | 1 | 1 | 2 | 1 | 1 | 1.2 | 0.2 |
| Mononuclear cells | 3 | 3 | 4 | 4 | 3 | 3.4 | 0.2 |
| Eosinophils | 2 | 2 | 3 | 3 | 2 | 2.4 | 0.2 |
| Edema | 2 | 2 | 3 | 3 | 2 | 2.4 | 0.2 |
| Epithelial hyperplasia | 2 | 2 | 3 | 3 | 2 | 2.4 | 0.2 |
| Fibrosis, subcuticular | 3 | 3 | 3 | 3 | 3 | 3.0 | 0.0 |
| *Total | 10 | 10 | 13 | 13 | 10 | | |
| *Mean Group Score | | | | | | 11.2 | 0.7 |

**Table 5: 6 week PBS control**

| Treatment Animal No. | Group 3B | | | | | | |
|---|---|---|---|---|---|---|---|
| | PBS wk6 | | | | | | |
| | 3-1 | 3-2† | 3-3† | 3-4† | 3-5 | Mean | SE |
| Inflammation | 2 | 1 | 1 | 1 | 2 | 1.4 | 0.2 |
| Neutrophils | 0 | 0 | 0 | 0 | 0 | 0.0 | 0.0 |
| Mononuclear cells | 2 | 1 | 1 | 1 | 2 | 1.4 | 0.2 |
| Eosinophils | 2 | 1 | 1 | 1 | 2 | 1.4 | 0.2 |
| Edema | 0 | 0 | 0 | 0 | 0 | 0.0 | 0.0 |
| Epithelial hyperplasia | 2 | 1 | 0 | 0 | 2 | 1.0 | 0.4 |
| Fibrosis, subcuticular | 0 | 1 | 0 | 0 | 0 | 0.2 | 0.2 |
| *Total | 4 | 3 | 1 | 1 | 4 | | |

| | | | | | | | |
|---|---|---|---|---|---|---|---|
| Codes and Symbols 0 = No Findings 1 = Minimal 2 = Mild 3 = Moderate 4 = Marked * = Excludes cellular inflammation component scores (neutrophils, mononuclear cells, eosinophils). † = Mixed cellular infiltrate and edema in subcutis | | | | | | | |

These results demonstrate that injection of purified TSLP into the skin of mice leads to sub-epithelial fibroblast accumulation and collagen deposition as early as two weeks post-injection. This response is increased over the six-week time course and was accompanied by observed skin thickening, edema, and significant cellular accumulation in the epidermis, dermis and subcutin. This response demonstrates the involvement of TSLP in the promotion of fibrotic disease.

In a follow-up experiment, five groups of 8-week-old Balb/c female mice (Charles River) were treated according to the following protocol. Each group contained 5 mice. The groups were injected intradermally on distinct parts of the back with 100 ul total volume as described above. Group 1 received one injection for one week (one injection total) of 10 ug MSA (mouse serum albumin, a negative control), 10 ug TSLP, and PBS on distinct parts of the back. Group 2 was injected once a week for two weeks (two injections total) with 10 ug MSA,10 ug TSLP, and PBS on distinct parts of the back. Group 3 was injected three times a week for two weeks (6 injections total) with 10 ug MSA, 10 ug TSLP, and PBS on distinct parts of the back. Group 4 was injected three times a week for two weeks (six injections total) with 1 ug MSA, 1 ug TSLP, and PBS on distinct parts of the back. Group 5 was injected 3 times a week for two weeks (six injections total) with 0.1ug MSA, 0.1ug TSLP, and PBS on distinct parts of the back. 72 hours after the final injection, the animals in each group were sacrificed, the skin was harvested, fixed in formalin, and made into slides for H&E staining for pathological evaluation. Evidence of subcuticular fibrosis from the skin samples was scored on a scale from 1 to 4. Fibrosis was visually scored based on fibroblast accumulation in the subcuticular region of the skin sections. The results are given in Figures 1 and 2. As seen in Figure 1, the single weekly dosage of TSLP for one week (Figure 1A, Group 1), or for two weeks (Figure 1B, Group 2) did not result in evidence of fibrosis. As seen in Figure 2A, the 10 ug dosage of TSLP administered three times a week for two weeks (Group 3) induced the greatest degree of fibrosis found in the skin, resulting a score of 3. As seen in Figure 2B, the 1 ug dosage of TSLP administered three times a week for two weeks (Group 4) resulted in a score of 2, and, as shown in Figure 2C, the 0.1 ug dosage of TSLP administered three times a week for two weeks (Group 5) resulted in a score of 1. The control MSA, and PBS alone did not induce any sign of fibrosis in the skin of the mice in any of the groups, with the exception of a single animal scoring a 1 for PBS in Group 4 (Figure 2C). This second experiment demonstrates that TSLP induces fibrosis in animals in a dose dependent manner.

## Claims

1. A thymic stromal lymphopoietin (TSLP) antagonist for use in reducing or preventing fibrosis in a subject suffering from a fibrotic disorder, wherein the antagonist:
a) binds to thymic stromal lymphopoietin or binds to thymic stromal lymphopoietin receptor; and
b) is selected from the group consisting of an antibody, a peptide or polypeptide.

2. A TSLP antagonist, for use as claimed in claim 1, which is selected from an anti-TSLP antibody, a soluble TSLP receptor, and a soluble IL-7 receptor/TSLP receptor heterodimer.

3. A thymic stromal lymphopoietin antagonist, for use as claimed in claim 1, which is an antagonist antibody selected from the group consisting of a human antibody, a humanised antibody, a single chain antibody, or an antibody binding fragment.

4. A thymic stromal lymphopoietin agent, for use as claimed in claim 1 or claim 2, wherein the peptide or polypeptide binding agent, soluble receptor or soluble heterodimer receptor further comprises an Fc domain.

5. A thymic stromal lymphopoietin antagonist, for use as claimed in claim 1, wherein the antagonist is a thymic stromal lymphopoietin receptor antagonist.

6. A thymic stromal lymphopoietin antagonist, for use as claimed in any one of claims 1 to 4, wherein,the fibrotic disorder is selected from the group consisting of scleroderma, interstitial lung disease, idiopathic pulmonary fibrosis, fibrosis arising from chronic hepatitis B or C, radiation-induced fibrosis, and fibrosis arising from wound healing.

7. A thymic stromal lymphopoietin antagonist, for use as claimed in any one of claims 1 to 4, for use with a second antagonist to a profibrotic cytokine, wherein the cytokine is selected from transforming growth factor β (TGF-β), interleukin-4 (IL-4), interleukin-5 (IL-5), interleukin-9 (IL-9), interleukin-13 (IL-13), granulocyte/macrophage-colony stimulating factor (GM-CSF), tumour necrosis factor alpha (TNF-α), interleukin-1 beta (IL-1β), connective tissue growth factor (CTGF), interleukin-6 (IL-6), oncostatin M (OSM), platelet derived growth factor (PDGF), monocyte chemotactic protein 1 (CCL2/MCP-1), and pulmonary and activation-regulated chemokine (CCL18/PARC).

8. A thymic stromal lymphopoietin antagonist, for use as claimed in any one of claims 1 to 7 in the form of a pharmaceutical composition, wherein the thymic stromal lymphopoietin antagonist is in admixture with a pharmaceutically acceptable carrier.

9. The use of an agent which reduces the amount or activity of thymic stromal lymphopoietin in tissue in the manufacture of a medicament for modulating fibroblast accumulation and collagen deposition in a subject suffering from a fibrotic disorder, wherein the agent is selected from the group consisting of an anti-TSLP antibody, a soluble TSLP receptor, a soluble IL-7 receptor/TSLP receptor heterodimer, an antisense oligonucleotide recognizing TSLP or TSLP receptor and interfering RNA.

10. The use of an agent as claimed in claim 9, wherein the amount of thymic stromal lymphopoietin or thymic stromal lymphopoietin receptor is reduced.

11. Use of a thymic stromal lymphopoietin antagonist in the manufacture of a medicament for reducing or preventing fibrosis in a subject suffering from a fibrotic disorder, wherein the antagonist:
a) binds to thymic stromal lymphopoietin or binds to thymal stromal lymphopoietin receptor; and
b) is selected from the group consisting of an antibody, a peptide or polypeptide.

## Patentansprüche

1. Thymusstroma-Lymphopoietin-Antagonist (TSLP-Antagonist) zur Verwendung beim Reduzieren oder Verhindern von Fibrose in einem Individuum, das an einer fibrotischen Störung leidet, wobei der Antagonist:
a) an Thymusstroma-Lymphopoietin bindet oder an einen Thymusstroma-Lymphopoietinrezeptor bindet und
b) aus der Gruppe ausgewählt ist, die aus einem Antikörper, einem Peptid oder einem Polypeptid besteht.

2. TSLP-Antagonist zur Verwendung nach Anspruch 1, der aus einem Anti-TSLP-Antikörper, einem löslichen TSLP-Rezeptor und einem löslichen IL-7-Rezeptor/TSLP-Rezeptor-Heterodimer ausgewählt ist.

3. Thymusstroma-Lymphopoietin-Antagonist zur Verwendung nach Anspruch 1, bei dem es sich um einen antagonistischen Antikörper handelt, der aus der Gruppe ausgewählt ist, die aus einem Humanantikörper, einem humanisierten Antikörper, einem einzelkettigen Antikörper oder einem Antikörper-Bindungsfragment besteht.

4. Thymusstroma-Lymphopoietin-Agens zur Verwendung nach Anspruch 1 oder Anspruch 2, wobei das Peptid- oder Polypeptid-Bindungsagens, der lösliche Rezeptor oder das lösliche Rezeptor-Heterodimer des Weiteren eine Fc-Domäne aufweist.

5. Thymusstroma-Lymphopoietin-Antagonist zur Verwendung nach Anspruch 1, wobei es sich bei dem Antagonisten um einen Antagonisten des Thymusstroma-Lymphopoietinrezeptors handelt.

6. Thymusstroma-Lymphopoietin-Antagonist zur Verwendung nach einem der Ansprüche 1 bis 4, wobei die fibrotische Störung aus der Gruppe ausgewählt ist, die aus Skleroderma, interstitieller Lungenkrankheit, idiopathischer Lungenfibrose, Fibrose infolge einer chronischen Hepatitis B oder C, strahlungsinduzierter Fibrose und Fibrose infolge von Wundheilung besteht.

7. Thymusstroma-Lymphopoietin-Antagonist zur Verwendung nach einem der Ansprüche 1 bis 4 zur Verwendung mit einem zweiten Antagonisten gegen ein profibrotisches Zytokin, wobei das Zytokin ausgewählt ist aus dem transformierenden Wachstumsfaktor β (TGF-β), Interleukin-4 (IL-4), Interleukin-5 (IL-5), Interleukin-9 (IL-9), Interleukin-13 (IL-13), dem Granulozyten/Makrophagenkolonie-stimulierenden Faktor (GM-CSF), Tumornekrosefaktor-alpha (TNF-α), Interleukin-1-beta (IL-1β), dem Bindegewebewachstumsfaktor (CTGF), Interleukin-6 (IL-6), Onkostatin M (OSM), dem von Thrombozyten stammenden Wachstumsfaktor (PDGF), dem chemotaktischen Monozyten-Protein 1 (CCL2/MCP-1) und dem pulmonalen und aktivierungsregulierten Chemokin (CCL18/PARC).

8. Thymusstroma-Lymphopoietin-Antagonist zur Verwendung nach einem der Ansprüche 1 bis 7 in der Form einer pharmazeutischen Zusammensetzung, wobei der Thymusstroma-Lymphopoietin-Antagonist einem pharmazeutisch zulässigen Träger beigemischt ist.

9. Verwendung eines Agens, welches die Menge oder Aktivität von Thymusstroma-Lymphopoietin in Gewebe reduziert, bei der Herstellung eines Medikaments zum Modulieren einer Fibroblastenansammlung und Kollagenablagerung in einem Individuum, das an einer fibrotischen Störung leidet, wobei das Agens aus der Gruppe ausgewählt ist, die aus einem Anti-TSLP-Antikörper, einem löslichen TSLP-Rezeptor und einem löslichen IL-7-Rezeptor/TSLP-Rezeptor-Heterodimer, einem Antisense-Oligonukleotid und interferierender RNA besteht, die TSLP oder den TSLP-Rezeptor erkennen.

10. Verwendung eines Agens nach Anspruch 9, wobei die Menge an Thymusstroma-Lymphopoietin oder Thymusstroma-Lymphopoietinrezeptor reduziert ist.

11. Verwendung eines Thymusstroma-Lymphopoietin-Antagonisten bei der Herstellung eines Medikaments zum Reduzieren oder Verhindern einer Fibrose bei einem Individuum, das an einer fibrotischen Störung leidet, wobei der Antagonist:
a) an Thymusstroma-Lymphopoietin bindet oder an einen Thymusstroma-Lymphopoietinrezeptor bindet und
b) aus der Gruppe ausgewählt ist, die aus einem Antikörper, einem Peptid oder einem Polypeptid besteht.

## Revendications

1. Antagoniste de la lymphopoïétine stromale thymique (TSLP) destiné à être utilisé pour réduire ou pour prévenir une fibrose chez un sujet atteint d'un trouble fibrotique, ledit antagoniste :
a) se liant à la lymphopoïétine stromale thymique ou se liant à un récepteur de la lymphopoïétine stromale thymique ; et
b) étant choisi dans le groupe constitué d'un anticorps, d'un peptide ou d'un polypeptide.

2. Antagoniste de la TSLP destiné à être utilisé selon la revendication 1, qui est choisi parmi un anticorps anti-TSLP, un récepteur soluble de la TSLP et un hétérodimère d'un récepteur soluble de l'IL-7 et d'un récepteur de la TSLP.

3. Antagoniste de la lymphopoïétine stromale thymique destiné à être utilisé selon la revendication 1, qui est un anticorps antagoniste choisi dans le groupe constitué d'un anticorps humain, d'un anticorps humanisé, d'un anticorps à chaîne unique, ou d'un fragment de liaison d'anticorps.

4. Agent de lymphopoïétine stromale thymique destiné à être utilisé selon la revendication 1 ou la revendication 2, dans lequel l'agent de liaison du peptide ou du polypeptide, le récepteur soluble ou l'hétérodimère soluble comprend en outre un domaine Fc.

5. Antagoniste de la lymphopoïétine stromale thymique destiné à être utilisé selon la revendication 1, ledit antagoniste étant un antagoniste des récepteurs de la lymphopoïétine stromale thymique.

6. Antagoniste de la lymphopoïétine stromale thymique destiné à être utilisé selon l'une quelconque des revendications 1 à 4, dans lequel le trouble fibrotique est choisi dans le groupe constitué de la sclérodermie, de la maladie pulmonaire interstitielle, de la fibrose pulmonaire idiopathique, de la fibrose résultant d'une hépatite B ou C chronique, de la fibrose radio-induite, et de la fibrose résultant d'une cicatrisation.

7. Antagoniste de la lymphopoïétine stromale thymique destiné à être utilisé selon l'une quelconque des revendications 1 à 4 et destiné à être utilisé avec un second antagoniste d'une cytokine profibrotique, dans lequel la cytokine est choisie parmi le facteur de croissance transformant bêta (TGF-β), l'interleukine 4 (IL-4), l'interleukine 5 (IL-5), l'interleukine 9 (IL-9), l'interleukine 13 (IL-13), le facteur de stimulation des colonies de granulocytes et de macrophages (GM-CSF), le facteur alpha de nécrose tumorale (TNF-α), l'interleukine 1 bêta (IL-1β), le facteur de croissance du tissu conjonctif (CTGF), l'interleukine 6 (IL-6), l'oncostatine M (OSM), le facteur de croissance dérivé des plaquettes (PDGF), la protéine chimiotactique des monocytes de type 1 (CCL2/MCP-1), et la chimiokine pulmonaire et régulée par activation (CCL18/PARC).

8. Antagoniste de la lymphopoïétine stromale thymique destiné à être utilisé selon l'une quelconque des revendications 1 à 7 sous forme de composition pharmaceutique, ledit antagoniste de la lymphopoïétine stromale thymique étant mélangé à un vecteur pharmaceutiquement acceptable.

9. Utilisation d'un agent qui réduit la quantité de lymphopoïétine stromale thymique ou son activité dans un tissu, dans la fabrication d'un médicament destiné à moduler l'accumulation de fibroblastes et les dépôts de collagène chez un sujet atteint d'un trouble fibrotique, ledit agent étant choisi dans le groupe constitué d'un anticorps anti-TSLP, d'un récepteur soluble de la TSLP, d'un hétérodimère d'un récepteur soluble de l'IL-7 et d'un récepteur de la TSLP, d'un oligonucléotide antisens reconnaissant la TSLP ou un récepteur de la TSLP, et de l'ARN interférent.

10. Utilisation d'un agent selon la revendication 9, dans lequel la quantité de lymphopoïétine stromale thymique ou de récepteur de la lymphopoïétine stromale thymique est réduite.

11. Utilisation d'un antagoniste de la lymphopoïétine stromale thymique dans la fabrication d'un médicament destiné à réduire ou à prévenir une fibrose chez un sujet atteint d'un trouble fibrotique, ledit antagoniste :
a) se liant à la lymphopoïétine stromale thymique ou se liant à un récepteur de la lymphopoïétine stromale thymique ; et
b) étant choisi dans le groupe constitué d'un anticorps, d'un peptide ou d'un polypeptide.
